# EUROPÄISCHE PATENTANMELDUNG

(11) **EP 4 728 960 A1**
(43) Veröffentlichungstag der Anmeldung: **22.04.2026**
(21) Anmeldenummer: 24206937.5
(22) Anmeldetag: 16.10.2024
(51) Int. Cl.: A61B 1/00, A61B 1/05, A61B 1/267, A61B 1/018, A61B 1/005

(54) **LARYNGOSKOPIESYSTEM ZUR DURCHFÜHRUNG EINES SICHEREN UND EINFACHEN EINFÜHRVORGANGS EINES MEDIZINISCHEN INSTRUMENTS IN EINEN PATIENTEN**

(71) Anmelder: Raymondos, Konstantinos, 30655 Hannover (DE); Kuper, Johannes H. M., 9561 JK Ter Apel (NL); Egges, Yolanda, 9561 JK Ter Apel (NL); Chi, Liguang, LangFang, Hebei Province 065201 (CN)
(72) Erfinder: Raymondos, Konstantinos, 30655 Hannover (DE); Kuper, Johannes H. M., 9561 JK Ter Apel (NL); Egges, Yolanda, 9561 JK Ter Apel (NL); Chi, Liguang, LangFang, Hebei Province 065201 (CN)
(74) Vertreter: Günther, Constantin

(57) **Zusammenfassung**

Die Erfindung betrifft ein Laryngoskopiesystem zur Durchführung eines sicheren und einfachen Einführvorgangs wenigstens eines medizinischen Instruments, das kein Endotrachealtubus ist, in einen Patienten. Das Laryngoskopiesystem weist als voneinander separate Komponenten wenigstens ein Video-Intubationslaryngoskop und das medizinische Instrument auf, z.B. einen Absaugkatheter oder ein Endoskop.

Außerdem betrifft die Erfindung das Gebiet des Intubierens eines Patienten mit einem Endotrachealtubus sowie weitere Eingriffe auf dem Gebiet der Laryngoskopie, z.B. zur Durchführung einer laryngoskopischen Untersuchung und/oder eines laryngoskopischen Eingriffs an einem Patienten.

## Beschreibung

Die Erfindung betrifft ein Laryngoskopiesystem zur Durchführung eines sicheren und einfachen Einführvorgangs wenigstens eines medizinischen Instruments, das kein Endotrachealtubus ist, in einen Patienten. Das Laryngoskopiesystem weist als voneinander separate Komponenten wenigstens ein Video-Intubationslaryngoskop und das medizinische Instrument auf, z.B. einen Absaugkatheter oder ein Endoskop.

Außerdem betrifft die Erfindung das Gebiet des Intubierens eines Patienten mit einem Endotrachealtubus sowie weitere Eingriffe auf dem Gebiet der Laryngoskopie, z.B. zur Durchführung einer laryngoskopischen Untersuchung und/oder eines laryngoskopischen Eingriffs an einem Patienten.

Laryngoskope gibt es in einer Vielzahl von verschiedenen Ausführungsformen. Eine Variante davon sind die sogenannten Intubationslaryngoskope, die als Hilfsmittel für die endotracheale Intubation dienen, d.h. zum sicheren Einführen eines Endotrachealtubus in die Luftröhre, z.B. für die Beatmung. Neuere Ausführungen von Intubationslaryngoskopen sind zum Teil als Video-Intubationslaryngoskope ausgebildet, die eine Bilderfassung und Bildanzeige der aufgenommenen Bilder ermöglichen. Ein solches Video-Intubationslaryngoskop ist z.B. aus der EP 4 371 467 A1 bekannt. Ferner sind Endoskope in der Medizintechnik als eine weitere Gruppe von medizinischen Geräten bekannt, mit denen das Innere von Organismen untersucht und manipuliert werden kann.

Die Aspiration von Mageninhalt stellt die häufigste aller schwerwiegenden Komplikationen bei der Atemwegssicherung dar. Sie kann zu einer sehr schwerwiegenden Lungenschädigung (ARDS) führen, die eine sehr hohe Mortalität von ca. 50% aufweist. Die einzige Möglichkeit, um z.B. aspirierte Magensäure aus den Bronchien wieder zu entfernen und somit eine Lungenschädigung effektiv zu verhindern oder zu vermindern, besteht in einer gezielten bronchoskopischen Absaugung. Dies sollte so schnell wie möglich nach Aspiration geschehen - am besten noch bevor die aspirierte Flüssigkeit durch den Beginn der Beatmung weiter in die Lunge gedrückt wird und dort das sehr empfindliche Lungengewebe schädigen kann.

Bei Notfallpatienten, die schon wegen eingeschränkter Schutzreflexe aspiriert haben und nur noch wenig oder gar nicht mehr atmen, wäre also die rasche Positionierung eines Bronchoskops in die Trachea mit sofortigem Absaugen der aspirierten Flüssigkeit wünschenswert. Am besten sollte das also noch vor oder fast zeitgleich mit der Intubation erfolgen, weil so dann auch das gleichzeitig erforderliche Absaugen und Beatmen erfolgen kann. Das ist aber von der Realität weit entfernt: Innerklinisch wird selbst bei Patienten mit erhöhtem Aspirationsrisiko erst dann ein Bronchoskop geholt und eingesetzt, wenn der Patient bereits aspiriert hat - es also schon relativ spät oder zu spät ist. Außerklinisch werden keine Bronchoskope auf Rettungsmitteln eingesetzt, da zur Steuerung die Expertise fehlt: Entsprechend schwerwiegend sind hier die Konsequenzen nach Aspiration, da eine Bronchoskopie nach Krankenhausaufnahme dann definitiv zu spät erfolgt. In der präklinischen Notfallrettung werden selbst bei bereits intubierten Patienten keine Bronchoskope auf Rettungsmitteln eingesetzt, da zur Steuerung die Expertise fehlt und selbst Einwegbronchoskope zu teuer sind: Entsprechend schwerwiegend sind hier die Konsequenzen nach Aspiration, da eine Bronchoskopie nach Krankenhausaufnahme dann definitiv zu spät erfolgt.

Das Einführen eines Bronchoskops in die Luftröhre erfordert ein entsprechendes Training und wird von manchen Anästhesisten wegen mangelnder Erfahrung nicht sicher beherrscht - obwohl es immer noch als Goldstandard für die Intubation bei erwartet schwierigem Atemweg gilt. Insbesondere bei komatösen bzw. sedierten Patienten treten hierbei erhebliche Probleme auf, da insbesondere Sekret und kollabierte obere Atemwege die Sicht und somit die Orientierung erschweren, was auch für die einfacher durchzuführende nasale Intubation gilt.

Verschiedene Atemwegshilfen bzw. sog. "oral airways" können das Einführen eines Bronchoskops um den Zungengrund herum erleichtern. Diese "oral airways" nach Williams, Berman oder Ovassapian sind teilweise oder über den gesamten Verlauf nach oben, zur Seite oder nach unten geöffnet, um Bronchoskop und Atemwegshilfe voneinander trennen zu können. Das wird besonders dann wichtig, wenn ein Endotrachealtubus über das in der Trachea positionierte Bronchoskop gefädelt werden soll, weil der Endotrachealtubus durch diese Atemwegshilfen nicht hindurch passt.

Allerdings kann bei der mit einem Bronchoskop durchgeführten Intubation die Tubuspassage am Kehlkopf nicht mehr gesehen und daher also auch nicht mehr gesteuert werden, da dann ja die Optik des Bronchoskops sich bereits in der Trachea befindet. Das kann dazu führen, dass der Tubus den Kehlkopfeingang nicht passieren kann und sogar zusammen mit dem Bronchoskop in die Speiseröhre fehlplatziert wird.

Wenn bereits das Einführen eines flexiblen mit einer Steuereinheit ausgestattetem Endoskops mit einem Videolaryngoskop für den ungeübten nur schwer oder nicht möglich ist, so gibt es noch mehr Schwierigkeiten bei dem Einführen von weichen, flexiblen medizinischen Instrumenten wie Schläuchen und/oder Kathetern. Solche Schläuche oder Katheter sind an ihrem vorderen (patientennahen) Abschnitt deswegen weich und somit flexibel, um Schäden an der sehr empfindlichen und sehr gut durchbluteten Schleimhaut von Trachea und Bronchien möglichst zu vermeiden. Wegen dieses weichen und flexiblen vorderen Abschnitts können solche Schläuche oder Katheter auch nicht ohne weitere Führungshilfsmittel in die Lunge eingeführt werden: Selbst das Einführen eines endotrachealen Beatmungstubus (Endotrachealtubus) mit einem stärker gekrümmten also hyperangulierten Spatel ist bereits ohne eine zusätzliche Hilfe wie ein Führungsstab, Metallstillet oder einen im Videolaryngoskop integrierten Führungsschacht nicht möglich.

In der klinischen Routine wird z.B. ein semirigider, bzw. halbstarrer, flexibler Führungsstab oder auch ein noch steiferer, aber immer noch flexibles Metallstilett in einen endotrachealen Beatmungstubus (Endotrachealtubus) eingeführt, um bei der videolaryngoskopischen Intubation um die natürliche Krümmung herum bis zum Kehlkopfeingang gelangen zu können. Wenn allerdings der vordere Abschnitt des Führungsstabes oder des Metallstillets nicht korrekt gebogen wird, so kann der Kehlkopfeingang trotz ausreichender videola-ryngoskopischer Sicht mit der Tubusspitze nur sehr schwer oder gar nicht erreicht werden. Leider passiert es im klinischen Alltag nur allzu oft, dass der Führungsstab beziehungsweise das Metallstillet in dem vorderen Bereich nicht korrekt gebogen wird. Dadurch wird eine videolaryngoskopische Intubation sehr schwer möglich bis zu unmöglich, da der Kehlkopfeingang zwar gesehen, aber nicht erreicht werden kann.

Auch mit einem korrekt gebogenen Führungsstab oder Metallstillett ist der Intubationsvorgang kompliziert: So muss nach Erreichen des Kehlkopfeingangs der Führungsstab oder das Metallstillett etwas zurückgezogen werden, damit der Endotrachealtubus um 90° nach unten in die Luftröhre eingeführt werden kann. Danach muss der Führungsstab oder das Metallstillett langsam weiter zurückgezogen werden, so dass die weiche Tubusspitze weiter eingeführt werden kann und durch die Festigkeit des Führungsstabes bzw. Metallstillets trotzdem weiter vorgeschoben werden kann, ohne sich im Rachen zu verbiegen, was wiederum zu einer Fehlintubation in die Speiseröhre führen kann. Diese Technik muss trainiert und auch zu zweit geübt werden, was im klinischen Alltag in der Regel zu selten vorkommt. Wenn allerdings in einer Notfallsituation bei nervösen und ungeübten Anwendern der Führungsstab beziehungsweise das Metallstillet nicht zurückgezogen, sondern weiter durch den Kehlkopf und dann in die Trachea vorgeschoben wird, besteht eine lebensbedrohliche Verletzungsgefahr.

Neben Endotrachealtuben werden auch Atemwegskatheter, Absaugschläuche oder Atemwegsdrähte in die Atemwege eingeführt. Atemwegskatheter oder Absaugschläuche unterscheiden sich vom Endotrachealtubus vor allem durch ihre Länge, da sie in der Regel tiefer in die Lunge, d. h. bis in die Bronchien, eingeführt werden. Absaugschläuche sind im Vergleich zu Atemwegskathetern sehr viel weicher, um die Verletzungsgefahr beim Einführen in die Atemwege zu minimieren. Atemwegsdrähte haben einen Durchmesser unter 1 mm, weisen kein Lumen auf und werden zur Schienung beziehungsweise als eine Art Sicherheitsanker in die Atemwege eingeführt (s.u.). Im allgemeinen Sprachgebrauch werden sowohl Absaugschläuche als auch Atemwegsdrähte als Atemwegskatheter bezeichnet.

Atemwegskatheter gibt es mit und ohne Lumen. In sehr spezialisierten Bereichen, beispielsweise bei der Versorgung von Neugeborenen oder von Patienten mit ausgeprägten Verengungen der oberen Atemwege, kommen auch dünne Atemwegskatheter mit einem Durchmesser von 1-4 mm zum Einsatz. Diese können mit einem biegsamen Führungsdraht im Lumen wie beim Endotrachealtubus gebogen werden. Auch hier gilt, dass beim Einführen des Atemwegskatheters der Führungsdraht langsam zurückgezogen werden muss. Andernfalls kann es zu lebensbedrohlichen Verletzungen innerhalb der Lunge kommen.

Über das Lumen eines Atemwegskatheters kann selbst bei einem Durchmesser von 1 mm nicht nur Sauerstoff verabreicht werden, sondern sogar eine komplette Beatmung erfolgen. Werden Atemwegskatheter ohne Führungsdraht als Intubationshilfe verwendet, sind sie ebenfalls halbstarr, so dass sie im vorderen Bereich eine Krümmung aufweisen, die das Erreichen des Kehlkopfeingangs ermöglicht. Auch wenn die Spitze des Atemwegskatheters zur Vermeidung von Verletzungen etwas weicher ist, so kann der Katheter ohne Verletzungsgefahr der Atemwege wegen dieser halbstarren Krümmung meistens nicht tiefer in die Lunge, also weder bis in den unteren Bereich der Luftröhre noch bis in den Bronchialbereich, geführt werden. Das kann nur mit weichen und somit flexiblen Atemwegskathetern erfolgen, deren vorderer Bereich sich nicht zu einer starren beziehungsweise halbstarren Krümmung verformen lässt.

Dementsprechend werden solche weichen Atemwegskatheter nur dann in die Luftröhre oder die Bronchien eingeführt, wenn die Führung durch den Kehlkopfbereich und in die Lunge hinein durch einen Endotrachealtubus ermöglicht wird. Im klinischen Alltag geschieht das beispielsweise, um einen Endotrachealtubus sicher zu entfernen oder auszutauschen: Der bis in die Bronchien eingeführte Atemwegskatheter soll also das erneute Einführen eines Endotrachealtubus sicher stellen. Sie werden als sogenannte Airway Exchange Catheter (AEC) bezeichnet. Mit ihrer Hilfe kann beispielsweise ein defekter Endotrachealtubus ausgetauscht werden.

Es gibt aber auch sehr dünne Atemwegskatheter mit einem Durchmesser von weniger als 1 mm, die kein Lumen aufweisen. Sie sind mit Führungsdrähten zur Positionierung von zentralvenösen Kathetern vergleichbar und können auch als Atemwegsdrähte bezeichnet werden. Auch diese Drähte sind wie die Führungsdrähte für zentralvenösen Katheter im vorderen Bereich sehr weich, um Verletzungen der sehr empfindlichen Atemwegsschleimhaut zu vermeiden. Bei krankhaften Verengungen der Atemwege, z.B. durch einen Tumor oder eine Schwellung, können diese Atemwegsdrähte durch den Arbeitskanal eines Bronchoskops bis in die Bronchien vorgeschoben werden. Nach Entfernung des Bronchoskops kann dann bei Bedarf z.B. bei weiterer Verengung der Atemwege versucht werden, einen Endotrachealtubus über diesen liegenden Atemwegsdraht in die Luftröhre einzuführen. Auch bei Patienten, die z.B. im Halsbereich in der Nähe der Atemwege oder an den Atemwegen selbst operiert wurden, kann ein solcher Atemwegsdraht bei wachen Patienten zur Sicherheit nach dem Entfernen des Endotrachealtubus in der Luftröhre verbleiben. Dies kann die erneute Intubation mit dem Endotrachealtubus erleichtern, wenn ein erhöhtes Risiko für eine Verlegung der Atemwege, z. B. durch Schwellungen nach einer Operation, besteht.

Es gibt auch sehr weiche Atemwegskatheter, die als Absaugschläuche verwendet werden: Sie sind länger als ein Endotrachealtubus und sie werden bis in den Bronchialbereich vorgeschoben, um Sekret aus den Atemwegen abzusaugen. Dies geschieht routinemäßig bei Patienten, die über einen Endotrachealtubus beatmet werden, da diese Patienten das Atemwegssekret nicht mehr abhusten können. Es gibt aber auch schwerkranke Patienten ohne Endotrachealtubus, die z.B. im Rahmen einer Lungenentzündung oder einer ausgeprägten Bronchitis das vermehrt produzierte Atemwegssekret nicht mehr aus den peripheren Lungenbereichen bis in die Luftröhre befördern und dann abhusten können. Solche Patienten, die eventuell über eine Maske nicht-invasiv beatmet werden, müssen dann unter Umständen nur deshalb endotracheal intubiert werden, um mit den flexiblen Absaugschläuchen in die Atemwege gelangen und das Sekret absaugen zu können.

Mit dem jetzigen Stand der Technik können also mit Standard-Videolaryngoskopen ohne Führungsschacht - insbesondere bei hyperanguliertem Spatel - Endotrachealtuben nur mit entsprechender Expertise und nur mit korrekt gebogenem Führungsstab oder Metallstillet in die Luftröhre eingeführt werden. Das bedeutet also, dass weder flexible Atemwegskatheter noch Atemwegsdrähte, noch weiche Absaugschläuche videolayngoskopisch über den Kehlkopf in die Luftröhre eingeführt werden können, da sie keinen Führungsstab oder Metallstillet aufweisen.

Dies gilt auch für Videolaryngoskope mit Führungsschacht, da der Führungsschacht nur für die Führung eines Endotrachealtubus mit entsprechendem Durchmesser ausgelegt ist. Bei kleineren Durchmessern, wie sie Atemwegskatheter, Absaugschläuche oder Atemwegsdrähte aufweisen, kann der Kehlkopf nicht oder nur sehr schwer erreicht werden: Der Spatel, den alle Videolaryngoskope mit Führungsschacht aufweisen, verhindert im Gegensatz zu der Führungsschiene des hier beschriebenen Larynkoskopiesystems, dass der Führungsschacht näher an den Kehlkopf herangeführt werden kann. Dies hat zur Folge, dass alle Katheter, Drähte und Schläuche mit kleinerem Durchmesser als für diesen Führungsschacht vorgesehen nicht in den Bereich des Kehlkopfes, sondern nach unten in die Speiseröhre geführt werden.

Im Gegensatz zu einem mit einer Steuereinheit ausgestatteten flexiblen Endoskop können also videolaryngoskopisch - insbesondere mit einem hyperanguliertem Spatel - Atemwegskatheter, Absaugschläuche oder Atemwegsdrähte nicht oder nur sehr schwer über den Kehlkopf in die Luftröhre und dann in die Bronchien eingeführt werden. Dies bedeutet eine entscheidende Einschränkung der Einsatzmöglichkeiten für Therapieoptionen, für die das einfache und schnelle Einführen von Atemwegskathetern, flexiblen Schläuchen wie z.B. Absaugschläuche und Atemwegsdrähten oder erforderlich ist:
Der Erfindung liegt die Aufgabe zugrunde, ein verbessertes Laryngoskopiesystem anzugeben, das bessere Möglichkeiten zur Untersuchung und/oder Behandlung eines Patienten bei vereinfachter Applizierbarkeit am Patienten ermöglicht.

Diese Aufgabe wird gelöst durch Laryngoskopiesystem zur Durchführung eines sicheren Einführvorgangs wenigstens eines medizinischen Instruments, das kein Endotrachealtubus ist, in einen Patienten, wobei das Laryngoskopiesystem als voneinander separate Komponenten wenigstens ein Video-Intubationslaryngoskop und das medizinische Instrument aufweist, mit folgenden Merkmalen:
a) das Video-Intubationslaryngoskop hat
   a1) einen Handgriff zum Halten des Video-Intubationslaryngoskops oder eines Teils davon und
   a2) eine mit dem Handgriff verbundene starre Führungsschiene, die in Längsrichtung einen gekrümmten Verlauf hat und einen in Längsrichtung von einem patientenfernen Ende bis zu einem patientennahen Ende der Führungsschiene verlaufenden, dem gekrümmten Verlauf folgenden Führungskanal hat, der zum Führen eines Endotrachealtubus für die Intubation eingerichtet ist,
   a3) wenigstens eine erste optische Erfassungseinrichtung im patientennahen Bereich an der Führungsschiene sowie
   a4) eine Videoanzeigevorrichtung zur Anzeige der von der wenigstens einen ersten optischen Erfassungseinrichtung aufgenommenen Bilder,
b) das medizinische Instrument hat einen flexiblen oder semirigiden länglichen Instrumentenkörper, insbesondere einen rohrförmigen oder schlauchförmigen Instrumentenkörper,
wobei der längliche flexible oder semirigide Instrumentenkörper in dem Führungskanal der Führungsschiene entlang des gekrümmten Verlaufs führbar ist und dabei einen entsprechend dem gekrümmten Verlauf der Führungsschiene ausgebildeten gekrümmten Verlauf annehmen kann, wobei der Instrumentenkörper über die gesamte Längserstreckung der Führungsschiene ohne Bedienung von Steuerungselementen in dem Führungskanal führbar ist und bei Platzierung des patientennahen Endes der Führungsschiene am Larynxeingang des Patienten unter visueller Kontrolle der Bilder der ersten optischen Erfassungseinrichtung durch einfaches Vorschieben wahlweise in den Larynxeingang, die Trachea, die Bronchien oder den Ösophagus des Patienten einführbar ist.

Eine bestimmte Ausführungsform der Erfindung, in der das medizinische Instrument ein Endoskop ist, betrifft ein Laryngoskopiesystem zur Durchführung eines sicheren Einführvorgangs wenigstens eines Endoskops in einen Patienten, wobei das Laryngoskopiesystem als voneinander separate Komponenten wenigstens ein Video-Intubationslaryngoskop und das Endoskop aufweist, mit folgenden Merkmalen:
a) das Video-Intubationslaryngoskop hat
   a1) einen Handgriff zum Halten des Video-Intubationslaryngoskops oder eines Teils davon und
   a2) eine mit dem Handgriff verbundene starre Führungsschiene, die in Längsrichtung einen gekrümmten Verlauf hat und einen in Längsrichtung von einem patientenfernen Ende bis zu einem patientennahen Ende der Führungsschiene verlaufenden, dem gekrümmten Verlauf folgenden Führungskanal hat, der zum Führen eines Endotrachealtubus für die Intubation eingerichtet ist,
   a3) wenigstens eine erste optische Erfassungseinrichtung im patientennahen Bereich an der Führungsschiene sowie
   a4) eine Videoanzeigevorrichtung zur Anzeige der von der wenigstens einen ersten optischen Erfassungseinrichtung aufgenommenen Bilder,
b) das Endoskop hat
   b1) als Instrumentenkörper einen flexiblen oder semirigiden länglichen rohrförmigen Endoskopkörper und b2) wenigstens eine zweite optische Erfassungseinrichtung am patientennahen Ende des rohrförmigen Endoskopkörpers,
wobei der längliche flexible oder semirigide Endoskopkörper in dem Führungskanal der Führungsschiene entlang des gekrümmten Verlaufs führbar ist und dabei einen entsprechend dem gekrümmten Verlauf der Führungsschiene ausgebildeten gekrümmten Verlauf annehmen kann, wobei der Endoskopkörper über die gesamte Längserstreckung der Führungsschiene ohne Endoskopsteuerung in dem Führungskanal führbar ist und bei Platzierung des patientennahen Endes der Führungsschiene am Larynxeingang des Patienten unter visueller Kontrolle sowohl der Bilder der ersten optischen Erfassungseinrichtung als auch der Bilder der zweiten optischen Erfassungseinrichtung wahlweise in den Larynxeingang, die Trachea, die Bronchien oder den Ösophagus des Patienten einführbar ist.

Beim erfindungsgemäßen Laryngoskopiesystem ist somit das medizinische Instrument bzw. dessen Instrumentenkörper hinsichtlich der Abmessungen und der Flexibilität bzw. Rigidität auf die Abmessungen und gekrümmte Formgebung der starren Führungsschiene abgestimmt, sodass der Instrumentenkörper ohne übermäßigen Kraftaufwand in dem gekrümmten Führungskanal entlanggeführt werden kann und daraus nicht unbeabsichtigt herausrutscht. Dies wird durch die Kanalform des Führungskanals und dessen gekrümmten Verlauf gefördert. Vorteilhafterweise muss dabei während des gesamten Einführvorgangs des Instrumentenkörpers keine Steuerung des Endoskops durch den Anwender durchgeführt werden, d. h. der Anwender ist von einer zusätzlichen manuellen Steuerung des Instrumentenkörpers entlastet.

Im Vergleich zur Führungsschiene, die aufgrund ihrer starren Ausführung im bestimmungsgemäßen Betrieb des Video-Intubationslaryngoskops zumindest im Wesentlichen nicht verformt werden kann, ist der flexible oder semirigide längliche Instrumentenkörper hinsichtlich seiner Flexibilität darauf ausgelegt, im bestimmungsgemäßen Betrieb verformt zu werden. Ein semirigider Instrumentenkörper ist dabei etwas steifer ausgebildet, zum Beispiel wie bei einem Atemwegskatheter, als ein flexibler Instrumentenkörper, wie zum Beispiel bei einem Bronchoskop.

Die Erfindung ermöglicht es auf diese Weise, dass ein medizinisches Instrument mit einem beliebigen flexiblen oder semirigiden länglichen z.B. massiven, rohrförmigen oder schlauchförmigen Instrumentenkörper einfach und ohne zusätzliche Hilfsgeräte in einen gewünschten Körperhohlraum wie die Trachea, die Bronchien oder den Ösophagus eines Patienten eingeführt werden kann. Soweit in dieser Anmeldung der Begriff "rohrförmig" verwendet wird, schließt dies auch eine schlauchförmige Ausführungsform mit ein. Die Erfindung ermöglicht es auch, dass mehrere medizinische Instrumente parallel bzw. gleichzeitig über die Führungsschiene in den Patienten eingeführt werden, d. h. durch den Führungskanal geschoben werden.

Dieses einfache Einführen des länglichen Instrumentenkörpers kann dabei insbesondere ohne zusätzliche Nutzung eines Endotrachealtubus erfolgen, d.h. ohne den Patienten intubieren zu müssen. Dabei können auf einfache Weise auch insbesondere solche Arten von länglichen Instrumentenkörpern eingeführt werden, die aufgrund ihrer Elastizität und/oder Flexibilität normalerweise nur mit anderen Hilfsmitteln, z.B. mittels des erwähnten Endotrachealtubus, eingeführt werden können. Dies wird durch die zuverlässige und starre Führung des länglichen Instrumentenkörpers in der starren Führungsschiene sowie der einfachen Platzierbarkeit des patientennahen Endes der starren Führungsschiene unmittelbar vor dem Larynxeingang möglich. Der längliche Instrumentenkörper kann z.B., wenn als medizinisches Instrument ein Endoskop verwendet wird, ein Endoskopkörper sein. Wenn als medizinisches Instrument ein Absaugkatheter verwendet wird, der zur Absaugung von Atemwegssekret dient, kann der längliche Instrumentenkörper ein Katheterkörper sein.

Wie erwähnt, ist das medizinische Instrument kein Endotrachealtubus, sondern ein sonstiges medizinisches Instrument, das den erwähnten flexiblen oder semirigiden länglichen Instrumentenkörper hat. In einer vorteilhaften Ausgestaltung kann dieser Instrumentenkörper weicher bzw. flexibler sein als ein Endotrachealtubus, d.h. er kann leichter verformt werden als ein Standard-PVC-Endotrachealtubus. In einer vorteilhaften Ausgestaltung ist der längliche Instrumentenkörper des medizinischen Instruments dazu eingerichtet, im Patienten über die Trachea hinaus bis zur Bifurkation oder bis in die Bronchien geführt zu werden. Im Gegensatz dazu ist ein Endotrachealtubus kürzer, hat einen größeren Durchmesser und ist ausschließlich dazu bestimmt, in die Luftröhre eingeführt zu werden, um eine Atemwegssicherung und Beatmung zu ermöglichen. Um jedoch weiter in die Lunge aus dem Trachealbereich hinaus in die Bronchien gelangen zu können, kann der längliche Instrumentenkörper länger als ein Endotrachealtubus sein, z.B. wenigstens 5 cm bzw. wenigstens 20 % oder wenigstens 30 % länger und/oder einen geringeren Durchmesser haben als ein für die entsprechende Patientengröße vorgesehener Endotrachealtubus, z.B. einen um wenigstens 20 % oder wenigstens 30 % geringeren Durchmesser.

Der flexible oder semirigide Instrumentenkörper kann über seine gesamte Längserstreckung oder Teile seiner Längserstreckung, z.B. im Bereich des patientennahen Endes, elastische und/oder plastisch verformbare Eigenschaften haben. Beispielsweise kann der Instrumentenkörper vom Anwender durch Zurechtbiegen plastisch in eine gewünschte Form gebogen werden. Im Fall elastischer Eigenschaften kann der Instrumentenkörper eine vordefinierte Form haben, z.B. geradlinig oder gekrümmt, in die er sich nach anderweitiger Verformung wieder selbsttätig zurückformt.

Wie oben bereits erwähnt, wird bei Patienten, die zum Beispiel Magensaft verschluckt - also aspiriert - haben, in der Regel zu spät gezielt unter endoskopischer Sicht abgesaugt: Es wird zunächst ein Endotrachealtubus eingeführt und erst dann wird - wenn überhaupt - ein flexibles Endoskop oder ein flexibler Absaugschlauch eingeführt. Für diese Intubation wird allerdings meistens eine Narkose durchgeführt, wobei spätestens dann aber beatmet werden muss. Diese Positivdruckbeatmung drückt jedoch die aspirierte Flüssigkeit weiter in die Bronchien in die Peripherie hinein, so dass sie nicht mehr abgesaugt werden und somit Lungengewebe schädigen kann. Das hat schwerwiegende Konsequenzen insbesondere bei der Notfallversorgung von Patienten. Wie oben bereits erwähnt, gehört die Aspiration von Mageninhalt in die Lunge zu den häufigsten schwerwiegenden, lebensbedrohlichen Komplikationen bei der Atemwegssicherung. Sie tritt im Krankenhaus mit einer Häufigkeit von über 1:500 auf und ist dahingegen bei der präklinischen Notfallrettung bis zu 100 Mal häufiger. Wenn bei Notfallpatienten eine Aspiration bereits bei Ankunft des Rettungspersonals vorliegt, so kann bei dem jetzigen Stand der Technik nicht vor der Platzierung eines Endotrachealtubus Flüssigkeit aus der Lunge abgesaugt werden. Dies hat wie bereits oben erwähnt sehr schwerwiegende Folgen. Ein frühzeitiges Absaugen von bereits aspiriertem Mageninhalt vor Beatmungsbeginn ohne ein Hineindrücken des Aspirats in die Lunge hinein, wäre ein entscheidender Vorteil.

Dies ist mit dem erfindungsgemäßen Laryngoskopiesystem erstmals möglich, da nun auch ein flexibler Absaugschlauch unabhängig von seinem Durchmesser sicher und einfach in die Trachea eingeführt werden kann. Da bei diesem erfindungsgemäßen Laryngoskopiesystem im Gegensatz zu allen anderen Videolaryngoskopen ein zusätzliches, sehr effektives Absaugsystem fest mit dem Laryngoskop verbunden werden kann, kann bereits im Rachen befindliche Flüssigkeit schnell und sicher abgesaugt werden, um eine freie Sicht auf den Kehlkopfeingang zu ermöglichen. Somit können erstmals auch flexible Absaugschläuche mit einem sehr großen Durchmesser von über 10 mm schnell und ohne ein Bronchoskop eingeführt werden. Dies ermöglicht ein sehr effektives Absaugen von größeren Flüssigkeitsmengen, selbst wenn dabei Partikel wie aspirierte Speisereste mit einem Durchmesser von mehreren Millimetern abgesaugt werden müssen, die normalerweise das Lumen herkömmlicher Absaugschläuche und natürlich auch das Lumen eines flexiblen Bronchoskops verstopfen würden. So große und somit so hocheffektive Absaugschläuche gibt es bisher nicht für die Atemwege, da Absaugschläuche, wie oben beschrieben, in den allermeisten Fällen nur über einen Endotrachealtubus eingeführt werden, der bei erwachsenen Patienten in der Regel einen Innendurchmesser von 6 bis 8 mm aufweist.

In ähnlicher Weise eröffnen sich durch die Erfindung auch neue therapeutische Möglichkeiten für Patienten, die ihr Atemwegssekret nicht ausreichend abhusten können und bei denen eine regelmäßige bronchoskopische Absaugung z.B. aufgrund mangelnder Expertise oder fehlender Ressourcen nicht möglich ist: Diese Patienten müssen nicht mehr endotracheal intubiert werden, nur um über einen Endotrachealtubus einen flexiblen Absaugschlauch bis in den Bronchialbereich einführen und das Sekret aus den Atemwegen absaugen zu können. Mit dem erfindungsgemäßen Laryngoskopiesystem ist es im Gegensatz zu allen anderen Videolaryngoskopen möglich, auch bei spontan atmenden, wachen Patienten einen im vorderen Bereich flexiblen Absaugschlauch sicher und einfach bis zum Kehlkopf und weiter bis tief in die Lunge in den Bronchialbereich einzuführen und Sekret sowohl im Notfall als auch in der Routineversorgung einfach und schnell abzusaugen. Somit können viele Patienten vor einer Intubation und der nachfolgenden Beatmung mit all seinen schwerwiegenden Folgen - wie die Schwächung der Atemmuskulatur und die Entwicklung einer Lungenentzündung - bewahrt werden.

Die Erfindung eignet sich auch für die Durchführung von Untersuchungen und/oder Eingriffen am Kehlkopf und im Kehlkopfbereich, zum Beispiel für die Stimmbandunterfütterung oder die Probenentnahme.

Flexible Schläuche können nicht nur zum Absaugen, sondern auch zur Verabreichung von Medikamenten in die Atemwege verwendet werden. Beispielsweise kann ein Lokalanästhetikum verabreicht werden, um die Luftröhre vor einer Intubation unter Spontanatmung lokal zu betäuben. Dadurch wird eine ausgeprägte Aktivierung der Schutzreflexe mit sehr starkem Husten und entsprechender Stressreaktion vermieden. Nach dem derzeitigen Stand der Technik muss bei Videolaryngoskopen ohne Führungsschacht ein Applikationskatheter verwendet werden, in den ein flexibler, halbstarrer Draht integriert ist. Dieser Draht muss sogar zweimal gebogen werden, damit einerseits der Applikationskatheter mit der ersten Biegung um den Zungengrund geführt werden kann und andererseits mit der zweiten Biegung das Lokalanästhetikum durch die Stimmritze gezielt in die Luftröhre appliziert werden kann. Je nach individueller Anatomie des Patienten sind diese Bögen jedoch nicht korrekt ausgebildet. Dies hat zur Folge, dass entweder der Kehlkopfeingang überhaupt nicht erreicht werden kann und/oder die Applikationsöffnung nicht in Richtung Luftröhre zeigt, sondern z.B. zu weit nach oben gegen die Stimmritze gerichtet ist. Daher muss dieser Applikationskatheter häufig wieder entfernt und eine oder sogar beide Biegungen korrigiert und an die individuelle Anatomie des Patienten angepasst werden.

Ein solcher flexibler Applikationskatheter mit integriertem halbstarren Führungsdraht kann jedoch bei Videolaryngoskopen mit Führungsschacht nicht verwendet werden, da er durch die Begrenzungen des Führungsschachtes nicht mehr steuerbar ist. Hier muss, wie bei dem erfindungsgemäßen Laryngoskopiesystem, ein flexibler Schlauch verwendet werden, der jedoch beim Stand der Technik nicht ohne Hilfsmittel bis zum Kehlkopfeingang geführt werden kann. Dies ist beim Stand der Technik nur möglich, wenn der flexible Schlauch in einen Endotrachealtubus eingeführt wird, dessen Durchmesser für dieses Gerät und damit für den Durchmesser des Führungskanals vorgesehen ist. Der Endotrachealtubus muss dann direkt bis vor den Kehlkopfeingang geführt werden, um mit dem kleineren flexiblen Schlauch nicht nach unten in die Speiseröhre, sondern durch die Stimmritze in die Luftröhre zu gelangen. Dies ist sehr aufwendig und umständlich und wird daher nicht oder nur sehr selten praktiziert.

Neben der Gabe eines Lokalanästhetikums können auch andere Medikamente in die Lunge verabreicht werden: So können Medikamente über einen flexiblen Schlauch gezielt in den Bronchialbereich appliziert werden. So kann z.B. Surfactant (surface active agent - eine grenzflächenaktive Substanz, die die Lungenbläschen auskleidet und stabilisert) ohne Bronchoskop gezielt in den Bronchialbereich verabreicht werden und damit die Entwicklung eines Lungenversagens frühzeitig gestoppt und eine Intubation mit all ihren Folgen vermieden werden. Auch andere Medikamente wie Antibiotika, Virostatika oder Antimykotika können mit Hilfe des erfindungsgemäßen Laryngoskopiesystems ohne Bronchoskopie und ohne Intubation über einen flexiblen Schlauch gezielt bis in den Bronchialbereich verabreicht werden. Im Gegensatz zur Verneblung über eine Gesichtsmaske können so Medikamente gezielt tief in die Lunge verabreicht werden. Dadurch wird vermieden, dass größere Mengen des Medikaments im Bereich der oberen Atemwege verbleiben und dort entsprechende Nebenwirkungen hervorrufen.

Schließlich können auch flexible Atemwegsdrähte sicher und einfach in der Luftröhre platziert werden. Solche Atemwegsdrähte, die im vorderen Bereich besonders flexibel und weich sind, werden, wie oben bereits erwähnt, bei Patienten eingesetzt, bei denen bereits eine Verengung der Atemwege vorliegt oder sich eine solche Verengung z.B. nach einer Operation oder Verletzung entwickeln kann. Über einen diesen Atemwegsdraht kann dann versucht werden, einen Atemwegskatheter oder einen Beatmungsschlauch sicherer in die Lunge "einzufädeln", wenn die Atemnot durch die zunehmende Verengung in solchen Fällen zunimmt.

Vorteilhafterweise kann das erfindungsgemäße Laryngoskopiesystem aufgrund der Ausgestaltung des Laryngoskops als Intubationslaryngoskop sowohl für die Intubation eines Patienten mit einem Endotrachealtubus als auch für die endoskopische Untersuchung mit dem Endoskop genutzt werden, wobei sogar beides gleichzeitig möglich ist oder zeitlich nacheinander, ohne dass die vom Arzt verwendeten Geräte getauscht werden müssen. Durch die Möglichkeit, anhand der Bilder der ersten optischen Erfassungseinrichtung und der Bilder der zweiten optischen Erfassungseinrichtung das Platzieren des patientennahen Endes der Führungsschiene an der richtigen Stelle am Larynxeingang visuell kontrollieren zu können und dabei zugleich das korrekte Vorschieben des Instrumentenkörpers in der Führungsschiene ebenfalls visuell kontrollieren zu können, werden sowohl eine sichere Intubation als auch ein schnelles und sicheres Platzieren des Instrumentenkörpers im Patienten gewährleistet. Im Ergebnis erlaubt das erfindungsgemäße Laryngoskopiesystem ein sicheres, einfaches Einführen des Endoskops in den Patienten unter kompletter Sichtkontrolle und ohne die Notwendigkeit der manuellen Steuerung des Endoskops.

Dabei kann die erste optische Erfassungseinrichtung dazu eingerichtet sein, einen am patientennahen Ende der Führungsschiene aus der Führungsschiene austretenden Bereichs des flexiblen oder semirigiden Instrumentenkörpers optisch erfassen zu können, sodass der Anwender auf der Videoanzeigevorrichtung auch die korrekte Platzierung des Instrumentenkörpers anhand der Bilder der ersten optischen Erfassungseinrichtung visuell kontrollieren kann. Dadurch, dass ein speziell für Intubationen optimiertes Laryngoskop, nämlich das Video-Intubationslaryngoskop, für das Einführen des Instrumentenkörpers genutzt wird, wird es möglich, auf einfache und schnelle Weise den Instrumentenkörper wahlweise in die Trachea oder den Ösophagus des Patienten einzuführen. Im Prinzip muss der Anwender dabei keine Manipulationsschritte am Instrumentenkörper vornehmen, sondern kann diesen einfach, wie bei einem Endotrachealtubus, in der Führungsschiene vorschieben. Dadurch, dass das patientennahe Ende der Führungsschiene direkt vor dem Larynxeingang platziert ist, ist ein korrektes Einführen des Instrumentenkörpers in die gewünschte Körperhöhle sichergestellt, auch ohne dass dabei eine Verstellung einer Krümmung des patientennahen Endes des Instrumentenkörpers erforderlich ist. Damit eignet sich das erfindungsgemäße Laryngoskopiesystem in besonderer Weise für Notfallsituationen bei Aspiration von Mageninhalt.

Durch die angezeigten Bilder der ersten optischen Erfassungseinrichtung kann der Anwender direkt kontrollieren, wenn der Instrumentenkörper oder der Endotrachealtubus am patientennahen Ende der Führungsschiene austritt. Die Führungsschiene kann vorteilhaft starr ausgebildet sein, sie kann somit im bestimmungsgemäßen Betrieb des Video-Intubationslaryngoskops zumindest im Wesentlichen nicht verformt werden. Der Führungskanal kann sich über die gesamte Länge der Führungsschiene erstrecken und dazu eingerichtet sein, eine Stützfläche für das hindurchgeführte medizinische Instrument, z.B. den Endotrachealtubus und/oder den länglichen Instrumentenkörper, in dem Führungskanal bereitzustellen, sodass das medizinische Instrument an dem Führungskanal anliegt.

Die erste optische Erfassungseinrichtung kann z.B. durch eine am patientennahen Ende der Führungsschiene angeordnete Kamera, eine Linse oder ein Objektiv gebildet sein. Wird zur optischen Erfassung ein durch die Führungsschiene verlegter Lichtleiter verwendet, so wird die am patientennahen Ende der Führungsschiene angeordnete Endseite des Lichtleiters als erste optische Erfassungseinrichtung angesehen. Um die Bilder der ersten optischen Erfassungseinrichtung zur Videoanzeigevorrichtung zu übertragen, kann z.B. eine an anderer Stelle angeordnete Kamera verwendet werden. Es ist vorteilhaft, die erste optische Erfassungseinrichtung nicht weiter als 15 mm entfernt vom patientennahen Ende der Führungsschiene anzuordnen. Dies unterstützt einen vorteilhaften Aufnahmewinkel und ermöglicht eine freie Sicht auf die Stimmbänder. Vergleichbares gilt für die zweite optische Erfassungseinrichtung. Die zweite optische Erfassungseinrichtung kann z.B. durch eine am patientennahen Ende des länglichen Instrumentenkörpers angeordnete Kamera, eine Linse oder ein Objektiv gebildet sein.

Gemäß einer vorteilhaften Ausführungsform der Erfindung ragt die Führungsschiene oder andere Elemente des Video-Intubationslaryngoskops, die starr mit der Führungsschiene verbunden sind, nicht vom patientennahen Ende der Führungsschiene in den Aufnahmebereich der ersten optischen Erfassungseinrichtung hinein. Dies hat den Vorteil, dass die Führungsschiene und ggf. die genannten anderen Elemente die freie Sicht der ersten optischen Erfassungseinrichtung im gesamten Aufnahmebereich nicht stören oder verdecken können.

Gemäß einer vorteilhaften Ausgestaltung der Erfindung ist vorgesehen, dass die Führungsschiene einen im Profil U-förmigen Führungskanal hat, der an einer beim Intubationsvorgang der Zunge des Patienten zugewandten Oberseite wenigstens teilweise offen ist, wobei der Führungskanal zumindest im patientennahen Bereich über wenigstens ein Abdeckelement an der Oberseite geschlossen ist. Dementsprechend ist der Führungskanal zumindest in patientennahen Bereich von allen Seiten geschlossen ausgebildet, was den Vorteil hat, dass der im Führungskanal geführte Gegenstand, z.B. der Instrumentenkörper, von allen Seiten geschützt ist und von der Umgebung abgeschirmt ist. Hierdurch wird eine sichere Führung des Instrumentenkörpers im Führungskanal sichergestellt. Das wenigstens eine Abdeckelement kann ein Teil der Führungsschiene sein, z.B. ein einstückig an der Führungsschiene angeformtes Abdeckelement. Das Abdeckelement kann auch ein separates Bauteil sein. Wie nachfolgend noch erläutert wird, kann das Abdeckelement auch durch einen Bereich eines Epiglottisheber gebildet sein.

Gemäß einer vorteilhaften Ausgestaltung der Erfindung ist vorgesehen, dass das Video-Intubationslaryngoskop einem Epiglottisheber zum Anheben der Epiglottis des Patienten hat, wobei der Epiglottisheber über wenigstens ein Lagerungselement an einem Bauteil des Video-Intubationslaryngoskops, insbesondere an der Führungsschiene, beweglich gelagert ist. Hiermit wird ein Intubationsgerät geschaffen, das ein zügiges, sicheres und patientenschonendes Intubieren auch unter Narkose erlaubt. Dabei kann insbesondere ein patientennaher Bereich des Epiglottishebers das zuvor erwähnte Abdeckelement zum Abdecken des Führungskanals an der Oberseite bilden.

Im unbetätigten Zustand des Epiglottishebers ist der Epiglottisheber zumindest im patientennahen Bereich auf der Oberseite der Führungsschiene angeordnet, z.B. indem er dort auf der Führungsschiene aufliegt. Vorteilhafterweise kann der Epiglottisheber an einem patientenfernen Ende ein Bedienelement zum manuellen Betätigen durch einen Anwender haben.

Allgemein kann gesagt werden, dass im unbetätigten Zustand des Epiglottishebers sich der patientennahe Bereich des Epiglottishebers näher an der Führungsschiene befindet als im betätigten Zustand. Wird der Epiglottishebers manuell betätigt, zum Beispiel durch Ziehen am Bedienelement, wird hierdurch der patientennahe Bereich des Epiglottishebers vom benachbarten Bereich der Führungsschiene fortgezogen.

Das Bedienelement kann insbesondere für die Aufnahme einer Zugkraft beim Betätigen des Epiglottishebers eingerichtet sein, insbesondere eine vom patientennahen Ende fortweisende Zugkraft. Der Anwender muss somit am Bedienelement eine Zugkraft aufbringen, um den Epiglottisheber zu betätigen.

Gemäß einer vorteilhaften Ausgestaltung der Erfindung ist vorgesehen, dass der Epiglottisheber einen überwiegend oder vollständig geschlossenen Bereich hat, in dem der Epiglottisheber entgegengesetzt U-förmig zur Führungsschiene ausgebildet ist. Hierdurch wird eine gute Stabilität und hohe Steifigkeit des Epiglottishebers erreicht. Zudem ist die Einheit aus der Führungsschiene und dem Epiglottisheber sehr flach bauend, so dass sie gut auch in enge Atemwege eingeführt werden kann. Auf diese Weise kann ein universelles Intubationsgerät geschaffen werden, das für alle Größen und Altersgruppen von Patienten geeignet ist, das heißt das sich für alle Größen von Atemwegen eignet (One size fits all).

Gemäß einer vorteilhaften Ausgestaltung der Erfindung ist vorgesehen, dass der Epiglottisheber zumindest in dem überwiegend oder vollständig geschlossenen Bereich die Führungsschiene beidseitig übergreift. Der Epiglottisheber ist somit zumindest in diesem Bereich breiter als die Führungsschiene. Dies erlaubt eine mechanisch einfache und zuverlässige Gleitlagerung des Epiglottishebers auf der Führungsschiene.

Gemäß einer vorteilhaften Ausgestaltung der Erfindung ist vorgesehen, dass der Epiglottisheber über das wenigstens eine Lagerungselement relativ zum Handgriff bewegbar ist, so dass durch eine Relativbewegung des Epiglottishebers gegenüber dem Handgriff die Epiglottis beim Intubationsvorgang anhebbar ist. Dies erlaubt eine ergonomisch günstige Betätigung. Die gesamte Einheit aus der Führungsschiene und dem Epiglottisheber kann vom Anwender mit einer Hand am Handgriff gehalten werden. Zum Betätigen des Epiglottishebers muss der Handgriff nicht in seiner Lage verändert werden, es ist lediglich eine manuelle Betätigung des Epiglottishebers am Bedienelement durch die andere Hand des Anwenders erforderlich.

Der Handgriff kann an der Unterseite der Führungsschiene angeordnet sein. Der Handgriff kann bezüglich der Unterseite der Führungsschiene im rechten Winkel abragen oder leicht in Richtung des patientennahen Endes geneigt und damit schräg zur Unterseite der Führungsschiene angeordnet sein, zum Beispiel in einem Winkel zwischen 60 und 90 Grad.

Gemäß einer vorteilhaften Ausgestaltung der Erfindung ist vorgesehen, dass die Führungsschiene zumindest in einem patientennahen Bereich einen Biegeabschnitt aufweist, in dem die Führungsschiene von der Seite aus betrachtet, an der der Handgriff angeordnet ist, konvex gebogen ist. Der U-förmige Führungskanal befindet sich somit auf der Innenseite des Biegeabschnitts. Der Biegeabschnitt kann z.B. einen 90 Grad-Bogen umfassen, oder einen Bogen von etwas mehr oder weniger als 90 Grad, z.B. etwa 80 Grad. Vorteilhaft ist z.B. ein Biegeabschnitt, der sich über einen Bogen von 70 bis 90 Grad erstreckt. Der Biegeabschnitt kann z.B. über einen Bogenwinkel von mindestens 80° denselben Biegeradius aufweisen. Dies ist förderlich für eine zielgerichtete, zügige Einführung der Führungsschiene in die Atemwege eines Patienten, so dass der Patient bestmöglich geschont wird. Durch die vorteilhafte Gestaltung der Führungsschiene kann insbesondere ein besserer Einführwinkel des Instrumentenkörpers in den Patienten geschaffen werden, sodass dieser beim Einführen nicht am Aryknorpel hängenbleibt.

Gemäß einer vorteilhaften Ausgestaltung der Erfindung ist vorgesehen, dass die Führungsschiene am patientenfernen Ende des Biegeabschnitts in einen linear verlaufenden oder im Vergleich zum Biegeabschnitt weniger stark gekrümmten Abschnitt übergeht. Dies ermöglicht insbesondere eine gleitende Linearführung oder quasi-lineare Führung des Epiglottishebers auf der Führungsschiene. Dabei kann die Führungsschiene im weniger stark gekrümmten Abschnitt einen wesentlich größeren Biegeradius haben als im Biegeabschnitt.

Gemäß einer vorteilhaften Ausgestaltung der Erfindung ist vorgesehen, dass der Führungskanal auf der Innenseite des Biegeabschnitts überwiegend oder vollständig offen ist. Auch dies ist förderlich für eine flachbauende Konstruktion des Intubationsgerätes. Der Führungskanal kann auf der Oberseite auch außerhalb des Biegeabschnitts überwiegend oder vollständig offen sein, auch über die gesamte Länge. Insbesondere kann die Führungsschiene zumindest im Biegeabschnitt oder insgesamt ein U-förmiges Profil haben.

Gemäß einer vorteilhaften Ausgestaltung der Erfindung ist vorgesehen, dass der Epiglottisheber sich Im Wesentlichen über die gesamte Längserstreckung der Führungsschiene erstreckt. Der Epiglottisheber kann auch am patientennahen Ende und/oder am patientenfernen Ende über die Führungsschiene hinaus ragen.

Gemäß einer vorteilhaften Ausgestaltung der Erfindung ist vorgesehen, dass der Epiglottisheber zumindest im patientennahen Bereich, insbesondere in einem Biegeabschnitt der Führungsschiene, die Führungsschiene oder zumindest den Führungskanal auf der Oberseite der Führungsschiene überwiegend oder vollständig geschlossen überdeckt. Der Epiglottisheber ist somit in dem Bereich, in dem er die Führungsschiene oder zumindest den Führungskanal auf der Oberseite der Führungsschiene überdeckt, überwiegend oder vollständig geschlossen ausgebildet und bildet hierdurch ein Dach über der wenigstens einen optischen Erfassungseinrichtung im patientennahen Bereich an der Führungsschiene und/oder der wenigstens einen optischen Erfassungseinrichtung im patientennahen Bereich an dem Epiglottisheber. Auf diese Weise ist zumindest die Erfassungsseite der ersten optischen Erfassungseinrichtung, z.B. eine Linse, ein Objektiv oder eine Kamera, unterhalb des überwiegend oder vollständig geschlossenen Bereichs des Epiglottishebers angeordnet (sozusagen unter dem Dach) und dadurch vor einer Sichtblockade durch Zungengewebe oder durch Verschmutzung geschützt.

Gemäß einer vorteilhaften Ausgestaltung der Erfindung ist vorgesehen, dass das Video-Intubationslaryngoskop eine Saugvorrichtung hat, die zum Absaugen von Flüssigkeiten im Bereich des patientennahen Endes der Führungsschiene eingerichtet ist, wobei die Saugvorrichtung wenigstens eine Ansaugöffnung zum Ansaugen der Flüssigkeiten hat. Eine solche Saugvorrichtung ermöglicht die Vermeidung einer Sichtblockade durch Flüssigkeiten und die Reinigung der Endoskopoptik durch Absaugen und ggf. Spülung. Die Saugvorrichtung kann mit der Führungsschiene verbunden sein, sodass der Anwender nicht zwei verschiedene Geräte separat handhaben muss, wie es im Fall eines separaten Saugers erforderlich wäre. Auch dies ist besonders vorteilhaft bei Anwendung des Laryngoskopiesystems für Notfallsituationen bei Aspiration von Mageninhalt.

Gemäß einer vorteilhaften Ausgestaltung der Erfindung ist vorgesehen, dass die Ansaugöffnung relativ zu einer beim Intubationsvorgang der Zunge des Patienten zugewandten Oberseite der Führungsschiene an einer Position unterhalb der wenigstens einen ersten optischen Erfassungsvorrichtung oder unterhalb der Führungsschiene angeordnet ist. Relativ zur Oberseite der Führungsschiene ist die wenigstens eine Ansaugöffnung somit tiefer als die erste optische Erfassungsvorrichtung bzw. benachbarte Bereiche der Führungsschiene gelegen, sodass die Absaugung am tiefstmöglichen Punkt erfolgen kann. Dementsprechend können auftretende Flüssigkeiten durch die Saugvorrichtung bereits abgesaugt werden, bevor sie die erste optische Erfassungsvorrichtung erreichen und dadurch die Sicht beeinträchtigen. Damit ist die erste optische Erfassungseinrichtung gut vor Verschmutzung geschützt. Die erfindungsgemäße Saugvorrichtung kann als Zusatzkomponente ausgebildet sein, die an dem Video-Intubationslaryngoskop befestigt werden kann. Die Anbringung der Saugvorrichtung am Video-Intubationslaryngoskop ist dabei optional und kann je nach Anwendungsfall durchgeführt werden oder entfallen. Die Saugvorrichtung kann auch baulich in das Video-Intubationslaryngoskop integriert sein, z.B. wenigstens zum Teil in die Führungsschiene integriert sein. Beispielsweise kann der wenigstens eine Saugkanal an der Führungsschiene angebracht sein oder innen in die Führungsschiene integriert sein.

Vorteilhafterweise kann die wenigstens eine Ansaugöffnung weiter vom patientenfernen Ende entfernt sein als die wenigstens eine erste optische Erfassungsvorrichtung.

Gemäß einer vorteilhaften Ausgestaltung der Erfindung ist vorgesehen, dass das Endoskop an seinem patientenfernen Ende einen Handgriff mit wenigstens einem Bedienelement hat, wobei das Endoskop einen Fernwirkmechanismus hat, über den durch Bedienung des wenigstens einen Bedienelements das patientennahe Ende des Instrumentenkörpers in unterschiedliche Krümmungen verstellbar ist. Auf diese Weise kann das Endoskop vom Anwender mittels des Bedienelements am Handgriff gesteuert werden. Vorteilhafterweise muss dieser Fernwirkmechanismus vom Anwender aber während des Einführvorgangs des Instrumentenkörpers über die Führungsschiene nicht betätigt werden, sodass der Anwender hiervon entlastet ist. Erst bei späteren Untersuchungsschritten kann zum Beispiel eine Steuerung der Krümmung des Instrumentenkörpers in der gewünschten Körperhöhle oder beim weiteren Vorschieben in dieser Körperhöhle durchgeführt werden. Der Fernwirkmechanismus kann eine rein mechanische Vorrichtung sein, z.B. unter Verwendung von Bowdenzügen oder vergleichbaren Steuerelementen.

Gemäß einer vorteilhaften Ausgestaltung der Erfindung ist vorgesehen, dass die Videoanzeigevorrichtung dazu eingerichtet ist, wenigstens zwei separate Videobildströme anzuzeigen, wobei die wenigstens zwei separaten Videobildströme die Bilder wenigstens zweier erster optischer Erfassungseinrichtungen des Video-Intubationslaryngoskops oder einer ersten optischen Erfassungseinrichtung des Video-Intubationslaryngoskops und einer zweiten optischen Erfassungseinrichtung des Endoskops umfassen. Auf diese Weise wird dem Anwender auf der Videoanzeigevorrichtung ein besonders guter Überblick über die jeweilige Situation des Einführvorgangs oder eines laryngoskopischen Eingriffs gegeben. Beispielsweise können durch zwei erste optische Erfassungseinrichtungen an der Führungsschiene dem Anwender Bildaufnahmen aus verschiedenen Perspektiven am patientennahen Ende der Führungsschiene angezeigt werden.

Gemäß einer vorteilhaften Ausgestaltung der Erfindung ist vorgesehen, dass die Videoanzeigevorrichtung dazu eingerichtet ist, die Anzeige wenigstens einen Videobildstroms automatisch in Abhängigkeit davon auf unterschiedliche Videobildquellen umzuschalten, ob eine zweite optische Erfassungseinrichtung des Endoskops mit der Videoanzeigevorrichtung verbunden ist oder nicht. Auf diese Weise wird der Anwender von der Notwendigkeit, manuelle Einstellungen vorzunehmen, entlastet. Das Laryngoskopiesystem erkennt automatisch, welche Art von Anzeigedarstellung durchgeführt werden soll. Beispielsweise kann die Videoanzeigevorrichtung dann, wenn eine zweite optische Erfassungseinrichtung des Endoskops nicht mit der Videoanzeigevorrichtung verbunden ist, die Bilder der wenigstens zwei ersten optischen Erfassungseinrichtungen des Video-Intubationslaryngoskops anzeigen. Wenn die zweite optische Erfassungseinrichtung mit der Videoanzeigevorrichtung verbunden ist, kann automatisch umgeschaltet werden auf eine Darstellung mit den Bildern einer ersten optischen Erfassungseinrichtung und einer zweiten optischen Erfassungseinrichtung.

Gemäß einer vorteilhaften Ausgestaltung der Erfindung ist vorgesehen, dass die Videoanzeigevorrichtung dazu eingerichtet ist, die Anzeige wenigstens einen Videobildstroms anhand einer Bedienung eines Bedienelements der Videoanzeigevorrichtung oder eines anderen Teils des Video-Intubationslaryngoskops zwischen verschiedenen Videobildquellen umzuschalten. Alternativ oder zusätzlich zur automatischen Umschaltung kann die Umschaltung der dargestellten Videobildströme somit auch durch manuelle Bedienung erfolgen, indem ein hierfür vorgesehenes Bedienelement betätigt wird. Als Bedienelement kann, wenn die Videoanzeigevorrichtung mit einem Touchscreen ausgebildet ist, eine Betätigung des Touchscreens verwendet werden, z.B. eine Wischbewegung.

Gemäß einer vorteilhaften Ausgestaltung der Erfindung ist vorgesehen, dass das Endoskop als Bronchoskop oder Gastroskop ausgebildet ist. Dementsprechend kann mittels des Bronchoskops eine Untersuchung der Atemwege und der Lunge erfolgen. Mittels des Gastroskops kann eine Untersuchung der Speiseröhre und des Magens erfolgen. Die Kombination von Bronchoskop mit einem Video-Intubationslaryngoskop vereinfacht das Einführen in die Trachea und auch die Tubuspassage, insbesondere bei schwierigen Atemwegen oder für Trainingszwecke. Diese Kombination ist selbst für eine nasale Intubation sinnvoll, was aber nur bei ausreichender Mundöffnung für das Einführen des Video-Intubationslaryngoskops möglich ist.

Gemäß einer vorteilhaften Ausgestaltung der Erfindung ist vorgesehen, dass das Laryngoskopiesystem als weitere Komponente einen Endotrachealtubus oder einen Atemwegskatheter hat, wobei der Instrumentenkörper in einem Lumen des Endotrachealtubus oder Atemwegskatheters platzierbar ist oder durch das Lumen hindurchführbar ist. Auf diese Weise kann eine endoskopische Untersuchung besonders effizient mit einer Intubation mit dem Endotrachealtubus oder Atemwegskatheter kombiniert werden. Dabei kann je nach Situation der Endotrachealtubus oder Atemwegskatheter vor dem Einführen des Instrumentenkörpers durch den Führungskanal geschoben werden und der Instrumentenkörper danach durch den Endotrachealtubus oder Atemwegskatheter hindurch geschoben werden. Alternativ kann der Endotrachealtubus oder Atemwegskatheter nach dem Einführen des Instrumentenkörpers durch den Führungskanal geschoben werden, indem der Endotrachealtubus oder Atemwegskatheter über den Instrumentenkörper geschoben wird. Alternativ kann der Endotrachealtubus oder Atemwegskatheter zusammen mit dem Instrumentenkörper als eine Einheit durch den Führungskanal der Führungsschiene geführt werden.

Das Video-Intubationslaryngoskop kann derart gestaltet sein, dass der Handgriff, die Führungsschiene, die wenigstens eine optische Erfassungseinrichtung sowie die Videoanzeigevorrichtung als eine gemeinsame Baueinheit ausgebildet sind. Beispielsweise kann die Videoanzeigevorrichtung als kleiner Bildschirm am Handgriff oder im Übergang vom Handgriff zur Führungsschiene angeordnet sein. Dies erlaubt eine direkte visuelle Kontrolle des Laryngoskopievorgangs durch Blick auf die in der Nähe des Handgriffs angebrachte Videoanzeigevorrichtung.

Gemäß einer vorteilhaften Ausgestaltung der Erfindung ist vorgesehen, dass der Handgriff, die Führungsschiene und die wenigstens eine erste optische Erfassungseinrichtung als eine gemeinsame Baueinheit ausgebildet sind, wobei die Videoanzeigevorrichtung als von dieser gemeinsamen Baueinheit getrennte Baueinheit ausgebildet ist, die zur Übertragung der von der wenigstens einen ersten optischen Erfassungseinrichtung aufgenommenen Bilder über wenigstens eine Datenübertragungsverbindung mit der wenigstens einen ersten optischen Erfassungseinrichtung verbindbar oder verbunden ist. Dies hat den Vorteil, dass die Videoanzeigevorrichtung baulich von der gemeinsamen Baueinheit (nachfolgend auch als Intubationsgerät bezeichnet), die den Handgriff, die Führungsschiene und die wenigstens eine optische Erfassungseinrichtung aufweist, getrennt ist und somit auch separat platziert werden kann. Auf diese Weise kann die Videoanzeigevorrichtung als wesentlich größerer Bildschirm ausgebildet sein als bei einer direkten Anbringung am Handgriff. Dementsprechend kann mehr Information sowie eine größere Bilddarstellung auf der Videoanzeigevorrichtung dargestellt werden. Zudem bewegt sich die Videoanzeigevorrichtung nicht bei den für den Laryngoskopievorgang erforderlichen Bewegungen der Führungsschiene mit. Die wenigstens eine Datenübertragungsverbindung kann eine drahtgebundene Datenübertragungsverbindung und/oder eine drahtlose Datenübertragungsverbindung drin, z.B. eine Bluetooth- oder WLAN-Verbindung.

In Jurisdiktionen, in denen auch medizinische Verfahren dem Patentschutz zugänglich sind, betrifft die Erfindung außerdem folgende Verfahren:
Verfahren zur Durchführung eines sicheren Einführvorgangs eines medizinischen Instruments, das kein Endotrachealtubus ist und das einen flexiblen oder semirigiden länglichen Instrumentenkörper hat, in einen Patienten unter Verwendung eines Video-Intubationslaryngoskops oder eines Laryngoskopiesystems der zuvor erläuterten Art, mit folgenden Schritten:
a) Einführen des Video-Intubationslaryngoskops mit dessen Führungsschiene durch den Mund des Patienten unter permanenter visueller Kontrolle der Bilder der ersten optischen Erfassungseinrichtung, bis das patientennahe Ende der Führungsschiene unmittelbar vor dem Larynxeingang platziert ist,
b) Hindurchführen des Instrumentenkörpers durch den Führungskanal der Führungsschiene unter permanenter visueller Kontrolle der Bilder der ersten optischen Erfassungseinrichtung,
c) Einführen des patientennahen Endes des Instrumentenkörpers wahlweise in die Trachea, die Bronchien oder den Ösophagus des Patienten unter weiterer permanenter visueller Kontrolle der Bilder der ersten optischen Erfassungseinrichtung, ohne dass dabei eine Verstellung einer Krümmung des patientennahen Endes des Instrumentenkörpers erforderlich ist.

Verfahren wie zuvor angegeben, dadurch gekennzeichnet, dass ein Endotrachealtubus vor dem Einführen des Instrumentenkörpers, nach dem Einführen des Instrumentenkörpers oder zusammen mit dem Instrumentenkörpers durch den Führungskanal der Führungsschiene geführt wird und mit dem patientennahen Ende vor dem Larynxeingang platziert wird.

Verfahren zur Durchführung eines sicheren Einführvorgangs eines Endoskops in einen Patienten unter Verwendung eines Video-Intubationslaryngoskops oder eines Laryngoskopiesystems der zuvor erläuterten Art, mit folgenden Schritten:
a) Einführen des Video-Intubationslaryngoskops mit dessen Führungsschiene durch den Mund des Patienten unter permanenter visueller Kontrolle der Bilder der ersten optischen Erfassungseinrichtung, bis das patientennahe Ende der Führungsschiene unmittelbar vor dem Larynxeingang platziert ist,
b) Hindurchführen des Endoskopkörpers durch den Führungskanal der Führungsschiene unter permanenter visueller Kontrolle der Bilder der ersten und der zweiten optischen Erfassungseinrichtung,
c) Einführen des patientennahen Endes des Endoskopkörpers wahlweise in die Trachea, die Bronchien oder den Ösophagus des Patienten unter weiterer permanenter visueller Kontrolle der Bilder der ersten und der zweiten optischen Erfassungseinrichtung, ohne dass dabei eine Verstellung einer Krümmung des patientennahen Endes des Endoskopkörpers erforderlich ist.

Verfahren wie zuvor angegeben, dadurch gekennzeichnet, dass ein Endotrachealtubus vor dem Einführen des Endoskopkörpers, nach dem Einführen des Endoskopkörpers oder zusammen mit dem Endoskopkörper durch den Führungskanal der Führungsschiene geführt wird und mit dem patientennahen Ende vor dem Larynxeingang platziert wird. Probleme und Nachteile, die bei Verwendung von Geräten nach dem bisherigen Stand der Technik auftreten, sind:
- Kombination von konventionellem Videolaryngoskop und flexiblem oder semirigidem Endoskop

Für die kombinierte Anwendung sind immer zwei Personen erforderlich: Die erste Person kann mit einer Hand das Videolaryngoskop halten und kann mit der zweiten Hand zwar das Bronchoskop einführen, aber nicht steuern. Für das Steuern eines flexiblen Bronchoskops sind immer zwei Hände erforderlich: Eine Hand hält und dreht das Bronchoskop und die zweite hält den Steuerungsteil, um dort die Bowdenzüge zur Steuerung der Bronchoskopspitze nach oben oder nach unten zu betätigen.

Die Durchführung einer simultanen Videolaryngoskopie und Bronchoskopie wäre also mit Geräten nach dem bisherigen Stand der Technik zur Zeit komplex und erfordert entsprechendes Training, was in der Praxis aber nur sehr selten erfolgt.

### - Kombination Videolaryngoskop ohne Führungsschacht und Bronchoskop

Bei den überwiegend verwendeten Videolaryngoskopen ohne Führungsschacht müsste das Bronchoskop ohne Führungshilfe von der Mundhöhle und dann um den Zungengrund bzw. den Spatel herum bis zum Larynxeingang geführt werden. Hierbei muss die Technik der Bronchoskopsteuerung sicher beherrscht werden, da die Bronchoskopspitze nach Überwindung der ersten Krümmung im Atemwegsverlauf nun mit einer Krümmung von ca. 90° in den Larynxeingang und in die Trachea gelenkt werden muss. Darüber hinaus öffnet der Spatel zwar die Atemwege, aber die Optik ist ungeschützt, so das Sekret, Blut oder ähnliches die Sicht blockieren kann.

### - Kombination Videolaryngoskop mit Führungsschacht und Bronchoskop

Bei den selten verwendeten Videolaryngoskopen mit Führungsschacht würde das Bronchoskop über den Führungsschacht geführt werden. Hierbei wäre es von den drei Seiten des Schachtes geschützt. Der Schacht führt das Bronchoskop allerdings nur bis zu seinem Ende. Wegen des Spatels des Videolaryngoskops kann das Schachtende des Führungsschachts nicht näher an den Larynxeingang herangeführt werden. Daher wird das Bronchoskop nach unten in die Speiseröhre geschoben, wenn man die Bronchoskopsteuerung nicht oder nicht korrekt betätigt.

Darüber hinaus ist die Bronchoskopführung mit dem Führungsschacht unsicher, da das Bronchoskop leicht an der seitlichen Öffnung des Schachtes hinausrutschen kann.

Probleme und Nachteile beim bisherigen Stand der Technik:
Da ohne Bronchoskopsteuerung die Luftröhre nicht mit flexiblen Endoskopen erreicht werden kann, gibt es bei einer kombinierten Anwendung von Videolaryngoskop und Bronchoskop folgende Probleme:
1. Es sind zwei Personen erforderlich, da die Bronchoskopsteuerung betätigt werden muss.
2. Auch mit Bronchoskopsteuerung ist das Einführen komplex, es muss erlernt und geübt werden.
3. Das Einführen eines Bronchoskops in die Lunge dauert lange, was die Notfallanwendung einschränkt.
4. Die Bronchoskopoptik ist ungeschützt, so dass die Sicht leicht blockiert werden kann.
5. Bei schachtgeführten Videolaryngoskopen kann das Bronchoskop leicht seitlich herausrutschen.
6. Durch diese Einschränkungen ist das Einführen von flexiblen Endoskopen ohne Steuerung nicht möglich.
7. Aus diesen Gründen erfolgt routinemäßig nach Aspiration kein frühes endoskopisch geführtes Absaugen, sondern -wenn überhaupt- erst nach Intubation und nach Beatmungsbeginn.

Lösungen für die oben aufgeführten sieben Problembereiche:
Durch die Kombination des eingangs beschriebenen, optimierten Video-Intubationslaryngoskops und des Endoskops, z.B. in Form eines Bronchoskops, kann die Luftröhre mit flexiblen Endoskopen auch ohne Steuerung erreicht werden, wodurch mittels der erfindungsgemäßen Laryngoskopiesystems die sieben Problembereiche wie folgt gelöst werden:
1. Es wird nur eine Person benötigt, da die Endoskopsteuerung nicht betätigt werden muss.
2. Auch ohne Endoskopsteuerung ist das Einführen sehr einfach und kann daher rasch erlernt werden.
3. Das Einführen eines Endoskops erfolgt sehr schnell, was die Notfallanwendung ermöglicht.
4. Die Endoskopoptik ist durch den Führungskanal und ggf. den Epiglottisheber und/oder die Saugvorrichtung geschützt, so dass die Sicht nicht leicht blockiert wird.
5. Das Endoskop kann nicht oder nur sehr schwer aus dem Führungskanal herausrutschen.
6. Durch diese Vorteile ist das Einführen von flexiblen Endoskopen auch ohne Steuerungseinheit möglich.
7. Aus diesen Gründen kann routinemäßig nach Aspiration ein frühes endoskopisch geführtes Absaugen vor Intubation und vor Beatmungsbeginn erfolgen.

Probleme und Nachteile beim bisherigen Stand der Technik beim Einführen eines Atemwegskatheters:
Da ohne eine zusätzliche Führung die Luftröhre nicht oder sehr schwer mit flexiblen Atemwegskathetern erreicht werden kann, gibt es bei einer kombinierten Anwendung von Videolaryngoskop und Atemwegskatheter folgende Probleme:
1. Da ein bereits platzierten Endotrachealtubus, ein Bronchoskop, ein Führungsstab, Metallstillet oder Führungsdraht erforderlich ist, um einen flexiblen Atemwegskatheter in die Lunge einzuführen, wird eine Notfallanwendung bei nicht intubierten Patienten unmöglich oder kaum möglich:
2. Ein Führungsdraht im Atemwegskatheter muss der Krümmung der Atemwege entsprechend gebogen werden, um den Kehlkopf zu erreichen, was sehr aufwändig ist, häufig nicht gelingt und daher eine Korrektur der Biegung erforderlich macht.
3. Das Einführen eines Atemwegskatheters mit gleichzeitigem Zurückziehen des Führungsdrahtes ist komplex und gefährlich, selbst wenn der Führungsdraht korrekt gebogen wurde und muss daher erlernt und geübt werden.
4. Da das Einführen eines flexiblen Atemwegskatheters mithilfe eines Führungsdrahtes sehr kompliziert ist, wird er meistens über einen bereits positionierten Endotrachealtubus eingeführt, was die Anwendungs- und Behandlungsmöglichkeiten sehr einschränkt.
5. Auch bei Videolaryngoskopen mit Führungskanal ist das Einführen eines flexiblen Atemwegskatheters ohne die zusätzliche Hilfe eines Endotrachealtubus nicht möglich.
6. Ein frühzeitiges Absaugen von aspirierten Flüssigkeiten, wie z.B. Mageninhalt, wird mit Atemwegskathetern bei Notfallpatienten vor der Intubation präklinisch derzeit nicht durchgeführt.
7. Ohne Führung durch einen Tubus ist auch das Absaugen von Atemwegssekret mit Hilfe eines Atemwegskatheters nicht oder nur sehr schwer möglich, weswegen Patienten intubiert und beatmet werden müssen.
8. Ohne Führung können auch keine flexiblen Atemwegskatheter in die Luftröhre bis in den Bronchialbereich eingeführt werden, um dort Medikamente, wie z.B. Lokalanästhetika oder Antibiotika zu verabreichen.
9. Das Einführen eines flexiblen Atemwegsdrahtes bei verengten Atemwegen kann nicht videolaryngoskopisch, sondern auch nur mit Hilfe eines bereits positionierten Endotrachealtubus oder über ein Bronchoskop erfolgen.
10. Aus diesen Gründen wird routinemäßig nach Aspiration oder bei viel Atemwegssekret nicht frühzeitig bei spontan atmenden Patienten mit einem Atemwegskatheter abgesaugt, sondern erst nach Intubation und Beatmungsbeginn.

Lösungen für die oben aufgeführten zehn Problembereiche:
Durch die Kombination des eingangs beschriebenen, optimierten Video-Intubationslaryngoskops und eines Atemwegskatheters oder vergleichbaren medizinischen Instruments kann die Luftröhre mit flexiblen Schläuchen auch ohne Führung durch ein Bronchoskop, Drähte oder Endotrachealtubus erreicht werden, wodurch mittels der erfindungsgemäßen Laryngoskopiesystems die zehn Problembereiche wie folgt gelöst werden:
1. Durch die sichere und einfache Führung mithilfe des Video-Intubationslaryngoskops wird erstmals eine Notfallanwendung von flexiblen Atemwegskathetern bei nicht intubierten Patienten ermöglicht.
2. Da keine Führungsdrähte mit dem Video-Intubationslaryngoskop erforderlich sind, müssen auch keine komplizierten Biegungen vorgenommen werden, was das Einführen eines flexiblen Atemwegskatheters erheblich erleichtert.
3. Da keine Führungsdrähte erforderlich sind, wird das Einführen eines flexiblen Atemwegskatheters auch dadurch erheblich vereinfacht, dass keine komplexen und darüber hinaus auch gefährlichen Verfahren erlernt und geübt werden müssen.
4. Das Einführen eines flexiblen Atemwegskatheters mit dem erfindungsgemäßen Laryngoskopiesystem ohne Führungsdraht ist deutlich einfacher und sicherer, so dass für eine Führung nicht erst intubiert werden muss, was die Anwendung von Behandlungsmöglichkeiten ganz erheblich erweitert:
5. Im Gegensatz zu anderen Videolaryngoskopen mit Führungskanal kann ein flexibler Atemwegskatheter ohne die zusätzliche Hilfe eines Endotrachealtubus in die Lunge eingeführt werden.
6. Mit dem erfindungsgemäßen Laryngoskopiesystem kann ein frühzeitiges Absaugen von aspirierten Flüssigkeiten, wie z.B. Mageninhalt, bei Notfallpatienten mit flexiblen Atemwegskathetern bereits vor der Intubation einfach durchgeführt werden.
7. Mit dem erfindungsgemäßen Laryngoskopiesystem kann ohne Führung durch einen Tubus das Absaugen von Atemwegssekret mit Hilfe eines flexiblen Atemwegskatheters bei nicht intubierten, spontan atmenden Patienten erfolgen, wodurch Intubation und Beatmung vermieden werden können.
8. Mit dem erfindungsgemäßen Laryngoskopiesystem können auch flexible Atemwegskatheter in die Luftröhre bis in den Bronchialbereich eingeführt werden, um dort Medikamente, wie z.B. Lokalanästhetika, Surfactant oder Antibiotika zu verabreichen. Hierdurch kann eine Intubation unter Spontanatmung vorbereitet werden oder frühzeitig der Krankheitsverlauf positiv beeinflusst und somit auch Intubation und Beatmung vermieden werden.
9. Mit dem erfindungsgemäßen Laryngoskopiesystem kann ein flexibler Atemwegsdraht bei verengten Atemwegen ohne Intubation und Bronchoskop in die Lunge geführt werden, was das Verfahren durch weniger Invasivität und vor allem durch Vereinfachung sehr viel sicherer macht.
10. Aus den genannten Lösungsansätzen kann mit dem erfindungsgemäßen Laryngoskopiesystem routinemäßig nach Aspiration oder bei viel Atemwegssekret frühzeitig bei spontan atmenden Patienten mit einem flexiblen Atemwegskatheter abgesaugt werden, wodurch Intubation und Beatmung vermieden werden können.

Im Sinne der vorliegenden Erfindung ist unter dem unbestimmten Begriff "ein" kein Zahlwort zu verstehen. Wenn also z.B. von einem Bauteil die Rede ist, so ist dies im Sinne von "mindestens einem Bauteil" zu interpretieren. Soweit Winkelangaben in Grad gemacht werden, beziehen sich diese auf ein Kreismaß von 360 Grad (360°).

Die Erfindung wird nachfolgend anhand von Ausführungsbeispielen unter Verwendung von Zeichnungen näher erläutert.

Es zeigen
- Figur 1: ein Laryngoskopiesystem,
- Figur 2: ein Intubationsgerät in Seitenansicht,
- Figur 3: ein Endoskop in perspektivischer Ansicht,
- Figur 4: ein Intubationsgerät in perspektivischer Ansicht,
- Figur 5: das Intubationsgerät gemäß Figur 4 mit einem Epiglottisheber in Seitenansicht,
- Figur 6: das Intubationsgerät gemäß Figur 4 mit einer Saugvorrichtung in perspektivischer Ansicht.

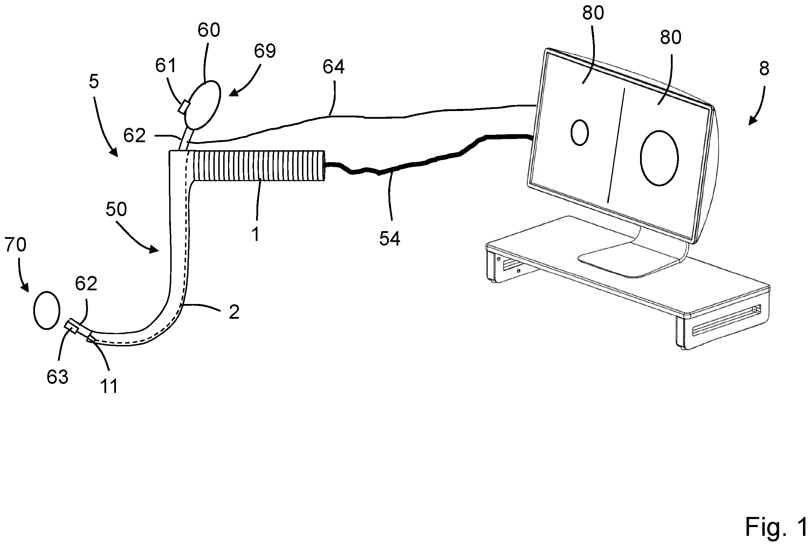

Das in Figur 1 dargestellte Laryngoskopiesystem weist ein Video-Intubationslaryngoskop 5, 8 auf, das in verschiedenen Baueinheiten gegliedert ist. Eine Baueinheit wird durch ein Intubationsgerät 5 gebildet, eine andere Baueinheit wird durch eine Videoanzeigevorrichtung 8 gebildet.

Das Intubationsgerät 5 hat einen Handgriff 1, der zum Halten des Intubationsgerät 5 durch den Anwender eingerichtet ist. Ferner hat das Intubationsgerät 5 eine mit dem Handgriff 1 verbundene starre Führungsschiene 2. Die Führungsschiene 2 hat in Längsrichtung einen gekrümmten Verlauf und einen in Längsrichtung von einem patientenfernen Ende bis zu einem patientennahen Ende der Führungsschiene verlaufenden, dem gekrümmten Verlauf folgenden Führungskanal, der zum Führen eines Endotrachealtubus für die Intubation eingerichtet ist. An einem patientennahen Ende der Führungsschiene 2 hat das Intubationsgerät 5 eine erste optische Erfassungseinrichtung 11, die fest mit der Führungsschiene 2 verbunden sein kann. Das Intubationsgerät 5 ist über eine Datenübertragungsverbindung 54 mit der Videoanzeigevorrichtung 8 verbunden.

Ferner hat das Laryngoskopiesystem ein medizinisches Instrument, das kein Endotrachealtubus ist. Beispielhaft wird hier als medizinisches Instrument ein Endoskop 69 beschrieben, die Erfindung kann ebenso mit anderen Arten medizinischen Instrumenten realisiert werden, z.B. mit einem Absaugkatheter zur Absaugung von Atemwegssekret.

Das Endoskop 69 hat einen flexiblen länglichen rohrförmigen Endoskopkörper 62. An einem patientenfernen Ende hat das Endoskop 69 einen mit dem rohrförmigen Endoskopkörper 62 verbundenen Handgriff 60 zum Halten des Endoskops 69. An dem Handgriff 60 ist wenigstens ein Bedienelement 61 angeordnet. Das Endoskop 69 hat ferner am patientennahen Ende des Endoskopkörpers 62 eine zweite optische Erfassungseinrichtung 63. Zudem kann das Endoskop 69 einen Fernwirkmechanismus haben, über den durch Bedienung des wenigstens einen Bedienelements 61 das patientennahe Ende des Endoskopkörpers 62 in unterschiedliche Krümmungen verstellt werden kann. Das Endoskop 69 kann über eine Datenübertragungsverbindung 64 mit der Videoanzeigevorrichtung 8 verbunden sein.

Die Figur 1 zeigt, dass das Endoskop 69 mit seinem Endoskopkörper 62 in der gekrümmten Führungsschiene 2 geführt ist, und zwar in einem Führungskanal der Führungsschiene 2, der nachfolgend noch näher erläutert wird. Durch die Führung in dem Führungskanal kann das patientennahe Ende des Endoskopkörpers 62 ebenso wie das patientennahe Ende der Führungsschiene 2 unter visueller Kontrolle in gewünschter Weise vor dem Larynxeingang 70 eines Patienten platziert werden. Die visuelle Kontrolle kann dabei anhand von auf der Videoanzeigevorrichtung 8 dargestellten, voneinander getrennten wenigstens zwei Videobildströmen 80, 81 erfolgen. Beispielsweise können über die getrennten Videobildströme 80, 81 die Bilder der ersten optischen Erfassungseinrichtung 11 an einer Seite und die Bilder der zweiten optischen Erfassungseinrichtung 63 an der anderen Seite dargestellt werden.

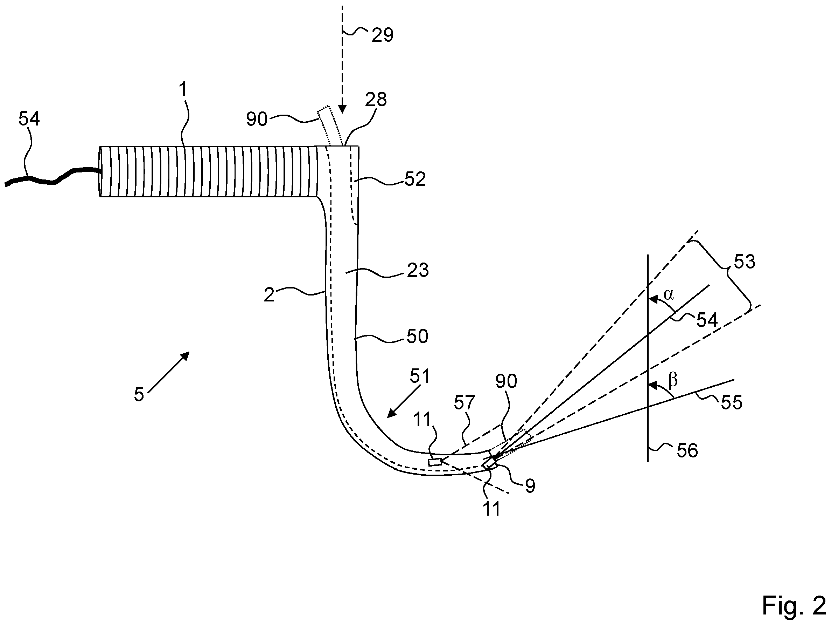

Die Figur 2 zeigt eine erste Ausführungsform eines Intubationsgeräts 5. Das Intubationsgerät 5 umfasst einen Handgriff 1, der von einem Bediener mit einer Hand, z. B. der linken Hand, gehalten werden kann. Mit dem Handgriff 1 ist eine Führungsschiene 2 fest verbunden, die ein schienenartiges Element sein kann. Die Führungsschiene 2 umfasst einen Führungskanal 23, der zum Führen eines Endotrachealtubus 90 geeignet ist. Der Führungskanal 23 kann die Form eines Kanals haben, was bedeutet, dass die Führungsschiene 3 eine Rückwand und eine linke und rechte Seitenwand umfasst. Die Führungsschiene 3 kann eine obere Wand umfassen, die sich auf der Innenseite des unteren gebogenen Abschnitts 51 der Führungsschiene 3 und auf der gegenüberliegenden Seite des Handgriffs 1 befindet. Eine obere Wand ist jedoch nicht in jedem Fall erforderlich, sodass die Führungsschiene 2 ohne obere Wand oder mit nur Teilabschnitten einer oberen Wand hergestellt werden kann. Das Profil der Führungsschiene 3 kann beispielsweise U-förmig oder abgerundet sein.

Die Führungsschiene 3 umfasst einen Eingang 28 für den Endotrachealtubus 90, der sich in der Nähe des Handgriffs 1 befindet. Der obere Teil der Führungsschiene 2, nahe dem Eingang 28, ist in einem Winkel von etwa 90° oder weniger als 90°zum Handgriff 1 angeordnet. Der Endotrachealtubus 90 ist im Bereich des Eingangs 28 und am patientennahen Ende 9 der Führungsschiene 2, wo der Endotrachealtubus 90 aus der Führungsschiene 2 austritt, teilweise gestrichelt dargestellt.

Die Führungsschiene 2 kann eine Einführstütze enthalten, falls die Führungsschiene 2 im Bereich nahe der Einführöffnung 28 keine obere Wand aufweist. Die Einführrichtung des Endotrachealtubus 9 wird durch einen gestrichelten Pfeil angezeigt. In Figur 2 wird die Einführstütze durch die teilweise obere Wand 52 gebildet. Die Einführstütze kann an der Führungsschiene 2 vorgesehen werden, um den Endotrachealtubus 90 zu stützen und zu zentrieren, wenn er in die Einführöffnung 28 eingeführt wird. Die Einführstütze kann die gesamte Breite des Führungskanals oder nur einen Teil der Breite abdecken. Eine Einführstütze, die nur einen Teil der Breite des Führungskanals 23 abdeckt, ermöglicht eine einfache Entfernung des Intubationsgeräts 5 vom Patienten, wenn der Endotrachealtubus 90 in seiner endgültigen Zielposition platziert ist, ohne dass das Risiko besteht, den Endotrachealtubus 90 aus seiner Position in der Luftröhre zu entfernen.

Die Führungsschiene 2 umfasst eine erste optische Erfassungseinrichtung 11 in der Nähe des patientennahen Endes 9 der Führungsschiene 2. Die erste optische Erfassungseinrichtung 11 kann in die Führungsschiene 2 integriert oder an der Führungsschiene 2 angebracht werden. Die Datenübertragungsverbindung 54 kann, wenn sie als elektrische Leitung ausgebildet ist, durch den Handgriff 1 geführt sein und in Form eines externen Verbindungskabels aus dem Handgriff 1 austreten.

Anstelle von Verbindungen über elektrische Leitungen kann eine drahtlose Schnittstelle für die Übertragung von Daten von der ersten optischen Erfassungseinrichtung 11 oder anderen elektrischen Geräten an externe Geräte verwendet werden. Beispielsweise kann eine Bluetooth-Schnittstelle verwendet werden. Das medizinische Gerät 1 kann mit einer integrierten elektrischen Stromquelle, wie einer Batterie, ausgestattet werden. In diesem Fall können elektrische Leitungen für die Stromversorgung vermieden werden. Dies verbessert die praktische Handhabung des Intubationsgeräts 5 weiter.

Die erste optische Erfassungseinrichtung 11 erfasst einen Bereich 53 vor dem patientennahen Ende 9 der Führungsschiene 2. Eine Mittellinie 54 des Erfassungsbereichs 53 ist in Figur 2 dargestellt. Außerdem zeigt eine Linie 55 eine hypothetische gerade Verlängerungslinie der Führungsschiene 2 von ihrem patientennahen Ende 9 aus. Außerdem ist eine vertikale Linie 56 parallel zur Einführrichtung 29 des Endotrachealtubus 90 in die Einführöffnung 28. Der Winkel α zwischen der Mittellinie 54 und der Linie 56 bzw. der Einführrichtung 29 ist kleiner als der Winkel β zwischen der Verlängerungslinie 55 und der Linie 56 bzw. der Einführrichtung 29. Der Winkel α kann zwischen 50 und 60° liegen. Außerdem tritt der Endotrachealtubus 90 aus der Führungsschiene 2 in einer Richtung aus, die einen kleineren Winkel als der Winkel β aufweist. Daher wird der Endotrachealtubus 90 in die Trachea geführt, die von der ersten optischen Erfassungseinrichtung 11 betrachtet und kontrolliert werden kann.

Wie in Figur 2 zu sehen ist, tritt der Endotrachealtubus 90 am patientennahen Ende 9 in einer Richtung innerhalb des Erfassungsbereichs 53 aus der Führungsschiene 2 aus. Die Austrittsrichtung des Endotrachealtubus 90 kann auf die Mittellinie 54 ausgerichtet werden. Zu diesem Zweck kann der Führungskanal 23 am patientennahen Ende 9 einen Rampenbereich aufweisen, der den Endotrachealtubus 90 beim Austritt aus der Führungsschiene 2 nach oben unterstützt, so dass der Endotrachealtubus 90 leicht in die Trachea eingeführt werden kann.

In einer vorteilhaften Ausführungsform ist die erste optische Erfassungseinrichtung 11 in der Mitte oder an einer Seite der Führungsschiene 2 so angeordnet, dass der Endotrachealtubus 90 in der Mitte des Erfassungsbereichs 53 erscheint, wenn der Endotrachealtubus 90 die Stimmbänder passiert.

Es kann eine weitere erste optische Erfassungseinrichtung 11 an der Führungsschiene 2 befestigt sein, z.B. neben der erstgenannten ersten optischen Erfassungseinrichtung 11. Die weitere erste optische Erfassungseinrichtung 11 hat einen Erfassungsbereich 57, der das patientennahen Ende 9 der Führungsschiene 2 und den aus der Führungsschiene 2 austretenden Endotrachealtubus 90 sowie einen umgebenden Bereich umfasst.

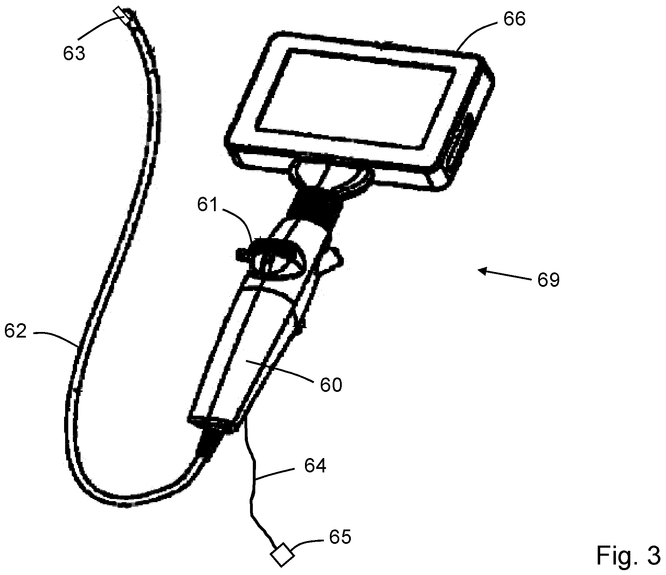

Die Figur 3 zeigt eine vorteilhafte Ausführung eines Endoskops 69, das z.B. als Bronchoskop ausgebildet sein kann. Wie erwähnt, hat das Endoskop 69 einen Handgriff 60 mit einem Bedienelement 61. Von dem Handgriff 60 ragt der flexible längliche rohrförmige Endoskopkörper 62 ab und erstreckt sich bis zu seinem patientennahen Ende, an dem am Endoskopkörper 62 die zweite optische Erfassungseinrichtung 63 angeordnet ist. Von dem Handgriff 60 zweigt zudem die Datenübertragungsverbindung 64 ab, z.B. in Form einer elektrischen Leitung mit einem Steckverbinder 65. Beim erfindungsgemäßen Laryngoskopiesystem können Endoskope verschiedenster Bauarten genutzt werden, sowohl Endoskope mit eigenem Bildschirm 66, auf dem die Bilder der zweiten optischen Erfassungseinrichtung 63 angezeigt werden, als auch Endoskope ohne eigenen Bildschirm.

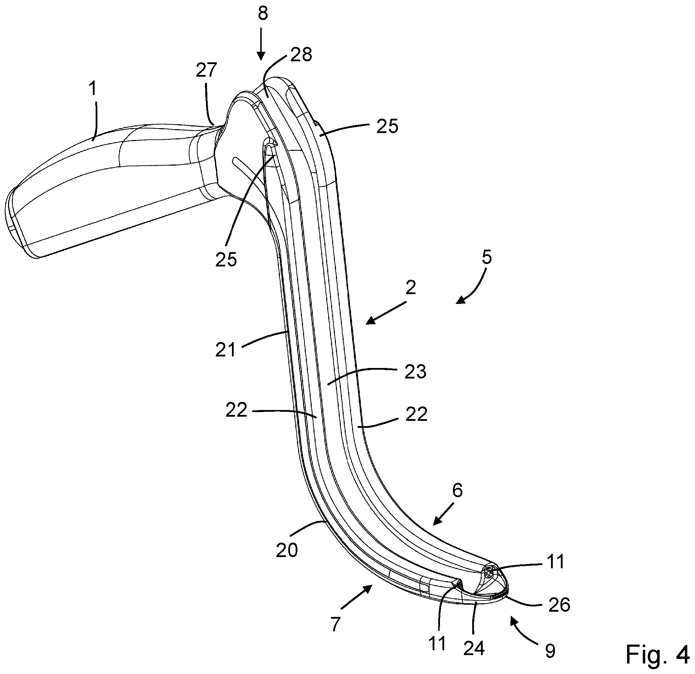

Die Figur 4 zeigt ein Intubationsgerät 5, das als Video-Intubationslaryngoskop ausgebildet ist. Das Intubationsgerät 5 hat einen Handgriff 1 zum Halten des Intubationsgeräts 5. Der Handgriff 1 kann besonders ergonomisch ausgeformt sein, z.B. mit einem entsprechenden Profil, das gut gegriffen werden kann und nicht so leicht verrutscht. Mit dem Handgriff 1 kann das Intubationsgerät 5 insbesondere während der Intubation geführt werden. Der Handgriff 1 bildet eine definierte Griffstelle zum Greifen und Halten des Intubationsgeräts 5. Der Handgriff 1 ist dazu eingerichtet, dass er vom Anwender mit der ganzen Hand gegriffen werden kann.

Das Intubationsgerät 5 hat außerdem eine starre Führungsschiene 2, die ebenfalls starr mit dem Handgriff 1 verbunden ist. Der Handgriff 1 ragt von einer Unterseite 7 der Führungsschiene 2 ab und bildet zu der Unterseite 7 der Führungsschiene 2 einen Winkel von weniger als 90°, z.B. im Bereich von 70°. Der Handgriff 1 ist auf diese Weise zum patientenfernen Bereich 8 der Führungsschiene 2 hin etwas schräggestellt. Die Führungsschiene 2 hat einen in Längsrichtung von dem patientenfernen Ende 8 bis zu dem patientennahen Ende 9 der Führungsschiene 2 verlaufenden Führungskanal 23 zum Führen des Endotrachealtubus 90.

Im patientennahen Bereich 9 endet die Führungsschiene 2 mit einem Biegeabschnitt 20, in dem die Führungsschiene 2 in Seitenansicht mit einem gewissen Radius gekrümmt ausgebildet ist. Nahe des freien Endes 24 der Führungsschiene 2, d.h. an der Spitze der Führungsschiene 2, ist wenigstens eine optische Erfassungseinrichtung 11 in die Führungsschiene 2 integriert. Beispielsweise kann nebeneinander auf gegenüberliegenden Seiten des Führungskanals 23 jeweils eine optische Erfassungseinrichtung 11 in die Führungsschiene 2 integriert sein. Der Biegeabschnitt 20 kann sich über einen Winkelbereich von z.B. 70° bis 100° erstrecken, wobei ein Winkel von etwas unter 90° besonders vorteilhaft ist. Vom patientennahen Bereich 9 aus gesehen schließt sich an den Biegeabschnitt 20 in der Seitenansicht gerade verlaufender Bereich 21 der Führungsschiene 2 an. Dieser gerade verlaufende Bereich 21 mündet in den Handgriff 1. Die Führungsschiene 2 ist im Querschnitt U-förmig ausgebildet und dementsprechend zur Oberseite 6 hin offen. Die Oberseite 6 der Führungsschiene 2 ist beim Intubationsvorgang der Zunge des Patienten zugewandt. Über die gesamte Längserstreckung der Führungsschiene 2 verläuft darin ein Führungskanal 23, in dem ein Endotrachealtubus 90 entlang der Führungsschiene 2 geführt werden kann. Der Führungskanal 23 ist links und rechts von erhabenen Randseiten der Führungsschiene 2 begrenzt.

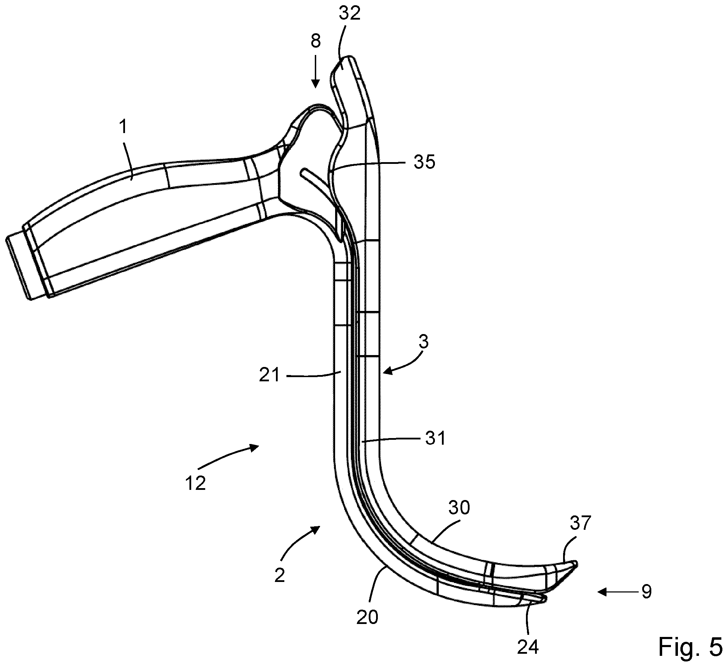

An der Oberseite 6 der Führungsschiene 2 kann ein Epiglottisheber 3 angeordnet werden, wie die Figur 5 zeigt. Die den Führungskanal 23 ist links und rechts begrenzenden erhabenen Randseiten der Führungsschiene 2 bilden mit Ihren zum Epiglottisheber 3 gewandten Oberflächen Auflagerungsflächen 22 zur Auflagerung des Epiglottishebers 3. Der Epiglottisheber 3 kann hinsichtlich seiner Formgebung an die Formgebung der Führungsschiene 2 angepasst sein, d.h. er folgt der Formgebung der Führungsschiene 2, sodass der Epiglottisheber 3 im gerade verlaufenden Bereich 21 der Führungsschiene 2 ebenfalls im Wesentlichen gerade verlaufend ausgebildet ist. Im Biegeabschnitt 20 der Führungsschiene 2 ist der Epiglottisheber 3 in analoger Weise gebogen ausgebildet. Erst am patientennahen freien Ende kann der Epiglottisheber 3 mit einer anderen Formgebung fortgeführt werden, insbesondere kann er über das freie Ende 24 der Führungsschiene 2 im patientennahen Bereich 9 etwas hinausstehen, z.B. indem dort eine Spatelspitze 37 ausgebildet ist. Am Intubationsgerät 5, insbesondere an der Führungsschiene 2, können formschlüssige Befestigungselemente 25 angeordnet sein, die mit formschlüssigen Befestigungselementen 35 des Epiglottishebers 3 zusammenwirken, um diesen an der Führungsschiene 2 zu halten.

Die Figur 7 zeigt das Intubationsgerät 5 mit einer daran befestigten Saugvorrichtung 4. Die Saugvorrichtung 4 hat einen Hauptkörper 41, der in seiner Längserstreckungsrichtung gekrümmt ausgebildet ist und insbesondere in diesem gekrümmten Bereich einem gekrümmten Verlauf der Führungsschiene 2 des Intubationsgeräts 5 folgt.

Der Hauptkörper 41 hat am patientennahen Ende 9 ein patientennahes Befestigungselement, mit dem der Hauptkörper 41 an einer Befestigungsstelle am patientennahen Ende 9 der Führungsschiene 2 anrastbar ist. Der Hauptkörper 41 hat außerdem ein patientenfernes Befestigungselement, mit dem der Hauptkörper 41 an einer zweiten Befestigungsstelle am patientenfernen Ende 8 des Intubationsgeräts 5, insbesondere an der Führungsschiene 2 und/oder dem Handgriff 1, befestigbar ist.

Am patientenfernen Ende 8 ist am Hauptkörper 41 eine Baugruppe 44 angeordnet, die mehrere Funktionen hat. So ist an der Baugruppe 44 an der zum patientennahen Ende 9 gewandten Seite das patientenferne Befestigungselement ausgebildet. Die Baugruppe 44 sorgt zudem für eine zusätzliche mechanische Stabilität des Hauptkörpers 41 im patientenfernen Bereich 8.

Zusätzlich ist an der Baugruppe 44 ein Anschluss 42 zum Anschluss eines Unterdruckschlauchs angeordnet. Durch den Unterdruckschlauch kann ein Unterdruck in das Innere der Baugruppe 44, die einen Hohlraum aufweist, eingeleitet werden. Die Baugruppe 44 ist über einen ersten Saugkanal und zusätzlich durch einen getrennt vom ersten Saugkanal ausgebildeten zweiten Saugkanal mit dem patientennahen Ende 9 des Hauptkörpers 41 verbunden. Die Saugkanäle enden mit einer jeweiligen Ansaugöffnung 40 in diesem patientennahen Bereich 9. Durch die Baugruppe 44 kann der über den Anschluss 42 eingeleitete Unterdruck beiden Saugkanälen zugeführt werden. Angesaugte Flüssigkeiten werden dann an den Ansaugöffnungen 40 angesaugt, durch die Saugkanäle zur Baugruppe 44 geführt und über den Anschluss 42 abgeführt.

An der Baugruppe 44 ist zudem ein Betätigungselement vorhanden, mit dem der Anwender mit einem Finger die Stärke der Absaugwirkung an den Ansaugöffnungen 40 beeinflussen kann. Das Betätigungselement kann beispielsweise als eine Öffnung mit definiertem relativ kleinem Querschnitt ausgebildet sein, die einen Zugang zum inneren Hohlraum in der Baugruppe 44 bereitstellt. Solange diese Öffnung mit dem Finger nicht abgedeckt ist, ist die Saugwirkung an den Ansaugöffnungen 40 relativ gering, da stattdessen Luft aus der Umgebung durch die Öffnung angesaugt wird. Wird die Öffnung mit dem Finger vollständig oder teilweise verschlossen, kann durch den Anwender auf diese Weise dosiert die Ansaugwirkung an den Ansaugöffnungen 40 erhöht und nach Wunsch variiert werden.

Vorteilhafter Weise sind die Saugkanäle baulich so in den Hauptkörper 41 integriert, dass sie nicht ungewollt komprimiert und dadurch verschlossen werden können. Dennoch verbleibt ausreichend Bauraum, um die Saugvorrichtung bzw. den Hauptkörper 41 an der Unterseite 7 der Führungsschiene 2 des Intubationsgeräts 5 anzurasten und dabei zumindest den unteren Teil der Führungsschiene 2 in dem U-förmigen Bereich des Hauptkörpers 41 aufzunehmen.

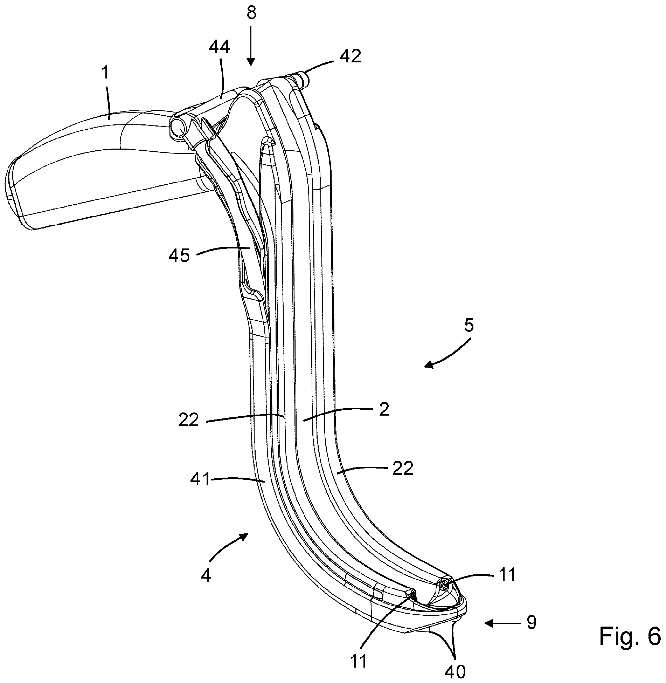

Man erkennt in der Figur 6 zudem gut die weit unten liegende Anordnung der Ansaugöffnungen 40, die unterhalb der optischen Erfassungseinrichtung 11 angeordnet sind.

## Patentansprüche

1. Laryngoskopiesystem zur Durchführung eines sicheren Einführvorgangs wenigstens eines medizinischen Instruments, das kein Endotrachealtubus (90) ist, in einen Patienten, wobei das Laryngoskopiesystem als voneinander separate Komponenten wenigstens ein Video-Intubationslaryngoskop (5, 8) und das medizinische Instrument (69) aufweist, mit folgenden Merkmalen:
a) das Video-Intubationslaryngoskop (5, 8) hat
a1) einen Handgriff (1) zum Halten des Video-Intubationslaryngoskops (5, 8) oder eines Teils davon und
a2) eine mit dem Handgriff (1) verbundene starre Führungsschiene (2), die in Längsrichtung einen gekrümmten Verlauf hat und einen in Längsrichtung von einem patientenfernen Ende (8) bis zu einem patientennahen Ende (9) der Führungsschiene (2) verlaufenden, dem gekrümmten Verlauf folgenden Führungskanal (23) hat, der zum Führen eines Endotrachealtubus (90) für die Intubation eingerichtet ist,
a3) wenigstens eine erste optische Erfassungseinrichtung (11) im patientennahen Bereich (9) an der Führungsschiene (2) sowie
a4) eine Videoanzeigevorrichtung (8) zur Anzeige der von der wenigstens einen ersten optischen Erfassungseinrichtung (11) aufgenommenen Bilder,
b) das medizinische Instrument (69) hat einen flexiblen oder semirigiden länglichen Instrumentenkörper (62), insbesondere einen rohrförmigen oder schlauchförmigen Instrumentenkörper,
wobei der längliche flexible oder semirigide Instrumentenkörper (62) in dem Führungskanal (23) der Führungsschiene (2) entlang des gekrümmten Verlaufs führbar ist und dabei einen entsprechend dem gekrümmten Verlauf der Führungsschiene (2) ausgebildeten gekrümmten Verlauf annehmen kann, wobei der Instrumentenkörper (62) über die gesamte Längserstreckung der Führungsschiene (2) ohne Bedienung von Steuerungselementen in dem Führungskanal (23) führbar ist und bei Platzierung des patientennahen Endes (9) der Führungsschiene (2) am Larynxeingang des Patienten unter visueller Kontrolle der Bilder der ersten optischen Erfassungseinrichtung (11) durch einfaches Vorschieben wahlweise in den Larynxeingang, die Trachea, die Bronchien oder den Ösophagus des Patienten einführbar ist.

2. Laryngoskopiesystem nach Anspruch 1, **dadurch gekennzeichnet, dass** das medizinische Instrument (69) ein Endoskop ist, das als Instrumentenkörper (62) einen flexiblen oder semirigiden länglichen rohrförmigen Endoskopkörper hat, wobei das Endoskop (69) wenigstens eine zweite optische Erfassungseinrichtung (63) hat, die am patientennahen Ende des rohrförmigen Endoskopkörpers (62) anordnet ist, wobei der längliche flexible oder semirigide Endoskopkörper (62) in dem Führungskanal (23) der Führungsschiene (2) entlang des gekrümmten Verlaufs führbar ist und dabei einen entsprechend dem gekrümmten Verlauf der Führungsschiene (2) ausgebildeten gekrümmten Verlauf annehmen kann, wobei der Endoskopkörper (69) über die gesamte Längserstreckung der Führungsschiene (2) ohne Endoskopsteuerung in dem Führungskanal (23) führbar ist und bei Platzierung des patientennahen Endes (9) der Führungsschiene (2) am Larynxeingang des Patienten unter visueller Kontrolle sowohl der Bilder der ersten optischen Erfassungseinrichtung (11) als auch der Bilder der zweiten optischen Erfassungseinrichtung (63) wahlweise in den Larynxeingang, die Trachea, die Bronchien oder den Ösophagus des Patienten einführbar ist.

3. Laryngoskopiesystem nach einem der vorhergehenden Ansprüche, **dadurch gekennzeichnet, dass** die Führungsschiene (2) einen im Profil U-förmigen Führungskanal (23) hat, der an einer beim Intubationsvorgang der Zunge des Patienten zugewandten Oberseite (6) wenigstens teilweise offen ist, wobei der Führungskanal (23) zumindest im patientennahen Bereich über wenigstens ein Abdeckelement an der Oberseite (6) geschlossen ist.

4. Laryngoskopiesystem nach einem der vorhergehenden Ansprüche, **dadurch gekennzeichnet, dass** das Video-Intubationslaryngoskop (5, 8) einem Epiglottisheber (3) zum Anheben der Epiglottis des Patienten hat, wobei der Epiglottisheber (3) über wenigstens ein Lagerungselement an einem Bauteil des Video-Intubationslaryngoskops (5, 8), insbesondere an der Führungsschiene (2), beweglich gelagert ist.

5. Laryngoskopiesystem nach Anspruch 4, **dadurch gekennzeichnet, dass** der Epiglottisheber (3) zumindest im patientennahen Bereich (8), insbesondere in einem Biegeabschnitt (20) der Führungsschiene (2), die Führungsschiene (2) oder zumindest den Führungskanal (23) auf der Oberseite (8) der Führungsschiene (2) überwiegend oder vollständig geschlossen überdeckt.

6. Laryngoskopiesystem nach einem der vorhergehenden Ansprüche, **dadurch gekennzeichnet, dass** das Video-Intubationslaryngoskop eine Saugvorrichtung (4) hat, die zum Absaugen von Flüssigkeiten im Bereich des patientennahen Endes (9) der Führungsschiene (2) eingerichtet ist, wobei die Saugvorrichtung (4) wenigstens eine Ansaugöffnung (40) zum Ansaugen der Flüssigkeiten hat.

7. Laryngoskopiesystem nach einem der vorhergehenden Ansprüche, **dadurch gekennzeichnet, dass** das medizinische Instrument (69) an seinem patientenfernen Ende einen Handgriff (60) mit wenigstens einem Bedienelement (61) hat, wobei das medizinische Instrument (69) einen Fernwirkmechanismus hat, über den durch Bedienung des wenigstens einen Bedienelements (61) das patientennahe Ende des Instrumentenkörpers (62) in unterschiedliche Krümmungen verstellbar ist.

8. Laryngoskopiesystem nach einem der vorhergehenden Ansprüche, **dadurch gekennzeichnet, dass** die Videoanzeigevorrichtung (8) dazu eingerichtet ist, wenigstens zwei separate Videobildströme (80) anzuzeigen, wobei die wenigstens zwei separaten Videobildströme (80) die Bilder wenigstens zweier erster optischer Erfassungseinrichtungen (11) des Video-Intubationslaryngoskops (5, 8) oder einer ersten optischen Erfassungseinrichtung (11) des Video-Intubationslaryngoskops (5, 8) und einer zweiten optischen Erfassungseinrichtung (63) des Endoskops (69) umfassen.

9. Laryngoskopiesystem nach Anspruch 8, **dadurch gekennzeichnet, dass** die Videoanzeigevorrichtung (8) dazu eingerichtet ist, die Anzeige wenigstens einen Videobildstroms (80) automatisch in Abhängigkeit davon auf unterschiedliche Videobildquellen umzuschalten, ob eine zweite optische Erfassungseinrichtung (63) des Endoskops (69) mit der Videoanzeigevorrichtung (8) verbunden ist oder nicht.

10. Laryngoskopiesystem nach Anspruch 8 oder 9, **dadurch gekennzeichnet, dass** die Videoanzeigevorrichtung (8) dazu eingerichtet ist, die Anzeige wenigstens einen Videobildstroms (80) anhand einer Bedienung eines Bedienelements der Videoanzeigevorrichtung (8) oder eines anderen Teils des Video-Intubationslaryngoskops (5, 8) zwischen verschiedenen Videobildquellen umzuschalten.

11. Laryngoskopiesystem nach einem der vorhergehenden Ansprüche, **dadurch gekennzeichnet, dass** das medizinische Instrument (69) als Absaugkatheter oder als Endoskop, insbesondere als Bronchoskop oder Gastroskop, ausgebildet ist.

12. Laryngoskopiesystem nach einem der vorhergehenden Ansprüche, **dadurch gekennzeichnet, dass** das Laryngoskopiesystem als weitere Komponente einen Endotrachealtubus (90) oder einen Atemwegskatheter hat, wobei der Instrumentenkörper (62) in einem Lumen des Endotrachealtubus (90) oder Atemwegskatheters platzierbar ist oder durch das Lumen hindurchführbar ist.

13. Laryngoskopiesystem nach einem der vorhergehenden Ansprüche, **dadurch gekennzeichnet, dass** der Handgriff (1), die Führungsschiene (2) und die wenigstens eine erste optische Erfassungseinrichtung (11) als eine gemeinsame Baueinheit ausgebildet sind, wobei die Videoanzeigevorrichtung (8) als von dieser gemeinsamen Baueinheit getrennte Baueinheit ausgebildet ist, die zur Übertragung der von der wenigstens einen ersten optischen Erfassungseinrichtung (11) aufgenommenen Bilder über wenigstens eine Datenübertragungsverbindung (54) mit der wenigstens einen ersten optischen Erfassungseinrichtung (11) verbindbar oder verbunden ist.
